# EUROPÄISCHE PATENTSCHRIFT

(11) **EP 1 300 135 B1**
(45) Veröffentlichungstag und Bekanntmachung des Hinweises auf die Patenterteilung: **03.05.2006**
(21) Anmeldenummer: 02021430.0
(22) Anmeldetag: 25.09.2002
(51) Int. Cl.: A61K 8/49, A61Q 5/10

(54) **Mittel zum Färben von keratinhaltigen Fasern enthaltend Derivate des 2-Arylidenindolin-3-ons**
Agent for dyeing keratinous fibres comprising 2-aryliden-indolin-3-one derivatives
Agent pour la teinture des fibres kératiniques comprenant des derivés de 2-arylidène indolin-3-one

(30) Priorität: 04.10.2001 DE 10148846
(43) Veröffentlichungstag der Anmeldung: 09.04.2003
(73) Patentinhaber: Henkel Kommanditgesellschaft auf Aktien, 40589 Düsseldorf (DE)
(72) Erfinder: Möller, Hinrich, Dr., 40789 Monheim (DE); Oberkobusch, Doris, Dr., 40591 Düsseldorf (DE); Höffkes, Horst, Dr., 40595 Düsseldorf (DE)

(56) Entgegenhaltungen:
- EP-A- 0 359 465
- DE-A- 4 335 623
- DE-A- 10 007 948
- US-A- 4 567 048
- US-A- 5 743 919
- DATABASE CA [Online] CHEMICAL ABSTRACTS SERVICE, COLUMBUS, OHIO, US; POMFRET, R. E.: "Some theoretical and practical viewpoints on the dyeing of polyester fibers with dispersion dyes" retrieved from STN Database accession no. 63:55189 XP002228403 & CAN. TEXTILE J. (1963), 80(20), 52-61 FROM: CZ 1964(35), ABSTR. NO. 2328.,

## Beschreibung

Die Erfindung betrifft ein Mittel zum Färben von keratinhaltigen Fasern, insbesondere menschlichen Haaren, das 2-Arylidenindolin-3-on-Derivate in Kombination mit ansgewähltem Verbindungen enthält, die Verwendung dieser Kombination in Mitteln zum Färben von keratinhaltigen Fasern sowie ein Verfahren zum Färben von keratinhaltigen Fasern, insbesondere menschlichen Haaren.

Für das Färben von keratinhaltigen Fasern kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, p-Phenylendiamin, 2,4-Diaminophenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorre-sorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch i.a. unter dem Einfluss von Oxidationsmitteln wie z. B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

Färbemittel mit 2-Arylidenindolin-3-on-Derivaten und deren Verwendung zum Färben von keratinhaltigen Fasern sind bislang nicht bekannt.

Aufgabe der vorliegenden Erfindung ist es, Färbemittel für Keratinfasern, insbesondere menschliche Haare, bereitzustellen, die hinsichtlich der Farbtiefe, der Grauabdeckung und den Echtheitseigenschaften qualitativ den üblichen Oxidationshaarfärbemitteln mindestens gleichwertig sind, ohne jedoch unbedingt auf Oxidationsmittel wie z. B. H₂O₂ angewiesen zu sein. Darüber hinaus dürfen die Färbemittel kein oder lediglich ein sehr geringes Sensibilisierungspotential aufweisen.

Überraschenderweise wurde nun gefunden, daß die in der Formel I dargestellten 2-Arylidenindolin-3-on-Derivate sich in Kombination mit nach folgend definierter Komponente B auch in Abwesenheit von oxidierenden Agentien hervorragend zum Färben von keratinhaltigen Fasern eignen. Sie ergeben Ausfärbungen mit hervorragender Brillanz und Farbtiefe und führen zu vielfältigen Farbnuan-cen. Es werden insbesondere Ausfärbungen über einen Nuancenbereich von gelb über gelbbraun, orange, braunorange, braun, rot, rotviolett bis hin zu blauviolett und schwarz erhalten. Der Einsatz von oxidierenden Agentien soll jedoch nicht prinzipiell ausgeschlossen werden.

Gegenstand der Erfindung ist ein Mittel zum Färben von keratinhaltigen Fasern, insbesondere menschlichen Haaren, enthaltend mindestens ein 2-Arylidenindolin-3-on-Derivat gemäß Formel I und/oder deren physiologisch verträglichen Salze, 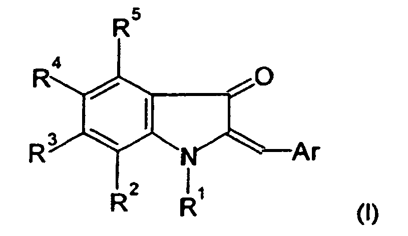 wobei
- R¹ steht für ein Wasserstoffatom, eine C₁-C₄-Alkylgruppe, eine C₂-C₆-Alkenylgruppe, eine Arylgruppe, eine Aryl-C₁-C₄-alkylgruppe, eine C₁-C₄-Acylgruppe, eine C₁-C₄₋Hydroxyalkylgruppe, eine C₂-C₄-Dihydroxyalkylgruppe, eine C₁-C₄-Sulfoalkylgruppe, eine C₁-C₄-Carboxyalkylgruppe, eine Gruppe R^{I}R^{II}N-(CH₂)ₘ-, worin R^{I} und R^{II} stehen unabhängig voneinander für ein Wasserstoffatom, eine C₁-C₄-Alkylgruppe, eine C,-C₄-Hydroxyalkylgruppe oder eine Aryl-C₁-C₄-alkylgruppe, wobei R^{I} und R^{II} gemeinsam mit dem Stickstoffatom einen 5-, 6- oder 7-gliedrigen Ring bilden können und m steht für eine Zahl 1, 2, 3 oder 4,
- R², R³, R⁴ und R⁵ stehen unabhängig voneinander für ein Wasserstoffatom, ein Halogenatom, eine C₁-C₄-Alkylgruppe, eine C₁-C₄-Alkoxygruppe, eine C₁-C₄₋Hydroxyalkoxygruppe, eine Hydroxygruppe, eine Nitrogruppe, eine Carboxygruppe, eine Sulfonsäuregruppe, eine Sulfamoylgruppe, eine Gruppe R^{III}R^{IV}N-(CH₂)_{q}-, worin R^{III} und R^{IV} stehen unabhängig voneinander für ein Wasserstoffatom, eine C₁-C₄₋Alkylgruppe, eine C₁-C₄-Hydroxyalkylgruppe oder eine Aryl-C₁-C₄-alkylgruppe und q ist steht für eine Zahl 0, 1, 2, 3 oder 4, wobei auch zwei der Reste R², R³, R⁴ und R⁵ zusammen mit dem Benzolring des 2-Arylidenindolin-3-on-Grundkörpers einen 5-oder 6-gliedrigen, carbozyklischen oder heterozyklischen, aliphatischen oder aromatischen Ring bilden können, und
- Ar steht für Phenyl, Naphthyl, Styryl, Pyridyl, Pyrimidyl, Pyrazyl, Pyrazidyl, Pyrrolyl, Furyl, Thienyl, 1,2,3-Triazinyl, 1,3,5-Triazinyl, Chinolyl, Isochinolyl, Indolyl, Indolinyl, 3-Oxo-indolinyl, Indolizinyl, Indanyl, Imidazolyl, 1,2,4-Triazolyl, 1,2,3-Triazolyl, Tetrazolyl, Benzimidazolyl, 1,3-Thiazolyl, Benzothiazolyl, Indazolyl, Benzoxazolyl, Chinoxalinyl, Chinazolinyl und Cinnolinyl, wobei der Ar-Rest gegebenenfalls durch ein Halogenatom, eine C₁-C₄-Alkylgruppe, eine C₁-C₄-Alkoxygruppe, eine C₁-C₄₋Hydroxyalkylgruppe, eine Hydroxygruppe, eine Carboxygruppe, eine Nitrogruppe, eine Sulfonsäuregruppe oder eine Gruppe R^{V}R^{VI}N-(CH₂)ᵣ-, worin R^{V} und R^{VI} stehen unabhängig voneinander für ein Wasserstoffatom, eine C₁-C₄-Alkylgruppe, eine C₁₋C₄-Hydroxyalkylgruppe oder eine Aryl-C₁-C₄-alkylgruppe und r steht für eine Zahl 0, 1, 2, 3 oder 4, substituiert ist.
und zusätzlich als Komponente B mindestens eine Verbindung, ausgewählt aus (a) Verbindungen mit primärer oder sekundärer Amino- oder Hydroxygruppe, ausgewählt aus aromatischen Hydroxyverbindungen, primären oder sekundären aromatischen Aminen und stickstoffhaltigen heterozyklischen Verbindungen, (b) Aminosäure und (c) CH-aciden Verbindungen.

Die Verbindungen mit der Formel I können auch in Form ihrer Hydrate vorliegen. Die Gegenionen der physiologisch verträglichen Salze der Verbindungen der Formel I, wie z.B. der Carboxylate und der Sulfonate, sind vorzugsweise Alkali-, Erdalkali- und Ammoniumionen. Enthalten die Verbindungen mit der Formel I N-Heteroaromaten oder Aminogruppen, können deren N-Atome quaterniert sein, z.B. durch eine Oxidogruppe oder durch C₁-C₄-Alkyl-, Aryl-C₁-C₄-alkyl-, C₁-C₄-Sulfoalkyl-, C₁-C₄-Carboxyalkyl-, C₁-C₄₋Hydroxyalkyl-, C₂-C₆-Alkenylgruppen, die ggf. substituiert sein können. In diesem Fall sind als Gegenionen der quaternierten heteroaromatischen Carbonylverbindungen Halogenide, wie Chlorid, Bromid oder lodid, Benzolsulfonat, p-Toluolsulfonat, C₁-C₄₋Alkansulfonat, wie Methansulfonat oder Ethansulfonat, Trifluormethansulfonat, Perchlorat, Sulfat, Hydrogensulfat, Tetrafluoroborat, Phosphat, Hexafluorophosphat oder Tetrachlorozinkat zu nennen.

Beispiele für die als Substituenten im Rahmen dieser Anmeldung genannten C₁-C₄₋Alkylreste sind die Gruppen Methyl, Ethyl, Propyl, Isopropyl, n-Butyl, sec-Butyl und tert.-Butyl. Ethyl und Methyl sind bevorzugte Alkylreste. Beispiele für bevorzugte C₂-C₆₋Alkenylreste sind Vinyl und Allyl. Erfindungsgemäß bevorzugte C₁-C₄-Alkoxyreste sind beispielsweise eine Methoxy- oder eine Ethoxygruppe. Weiterhin können als bevorzugte Beispiele für eine C₁-C₄-Hydroxyalkylgruppe eine Hydroxymethyl-, eine 2-Hydroxyethyl-, eine 2-Hydroxypropyl, eine 3-Hydroxypropyl- und eine 4-Hydroxybutylgruppe genannt werden. Eine 2-Hydroxyethylgruppe ist besonders bevorzugt. Beispiele für eine C₂-C₆₋Dihydroxyalkylgruppe ist die 2,3-Dihydroxypropylgruppe. Eine bevorzugte Hydroxy-C₁₋C₄-Alkoxygruppe ist die 2-Hydroxyethoxygruppe. Bevorzugte Arylgruppen sind Phenyl, Naphthyl und Biphenyl. Beispiele für Halogenatome sind F-, Cl-, oder Br-Atome, wobei Cl-Atome ganz besonders bevorzugt sind. Bevorzugte C₁-C₄-Aminoalkylgruppen sind die

Aminomethyl-, die Aminoethyl und die Aminopropylgruppe. Bevorzugte C₁-C₄₋Acylgruppen sind Formyl, Acetyl, Propionyl und Butyryl. Eine bevorzugte C₁-C₄₋Carboxyalkylgruppe ist die Carboxymethylgruppe. Beispiele für eine Aryl-C₁-C₄₋alkylgruppe sind Benzyl und 2-Phenylethyl. Die Aminomethyl-, 2-Aminoethyl-, 3-Aminopropyl-, 2-Dimethylaminoethyl-, Diethylaminomethyl-, Dimethylaminomethyl, 2-Methylaminoethyl-, Dimethylamino-, Piperidinomethyl-, Pyrrolidinomethyl, Morpholinomethyl- und die Aminogruppe sind Beispiele für eine Gruppe R'R"N-(CH₂)ₙ-, wobei die Diethylaminomethyl-, Piperidinomethyl, 2-Dimethylaminoethyl-, Dimethylamino- und die Aminogruppe besonders bevorzugt sind. Die weiteren verwendeten Begriffe leiten sich erfindungsgemäß von den hier gegebenen Definitionen ab. Die oben genannten Beispiele der verwendeten Reste sind somit auch für die folgenden Formeln II und III relevant.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyl- oder Acetylcellulose und synthetischer Fasern, wie z. B. Polyamid-, Polyacrylnitril-, Polyurethan- und Polyesterfasern verwendet werden.

2-Arylidenindolin-3-on-Derivate der Formel I sind zum großen Teil literaturbekannt, im Handel erhältlich oder nach bekannten Syntheseverfahren herstellbar.

Vorzugsweise sind die 2-Arylidenindolin-3-on-Derivate mit der Formel I ausgewählt aus 2-Benzyliden-indolin-3-on, 2-(2-Hydroxybenzyliden)-indolin-3-on, 2-(4-Hydroxybenzyliden)indolin-3-on, 2-(4-Methoxybenzyliden)-indolin-3-on, 2-(4-Methoxybenzyliden)-5-nitroindolin-3-on, 2-(4-Nitrobenzyliden)-4-nitro-indolin-3-on, 2-(4-Nitrobenzyliden)-5-methoxyindolin-3-on, 2-(3-Nitrobenzyliden)-indolin-3-on, 2-(2-Nitrobenzyliden)-indolin-3-on, 1-Methyl-2-(4-nitrobenzyliden)-indolin-3-on, 1-Methyl-2-(3-Nitrobenzyliden)-indolin-3-on, 1-Methyl-2-(2-nitrobenzyliden)-indolin-3-on, 2-(3,4-Dimethoxybenzyliden)-indolin-3-on, 2-(3,4-Methylendioxybenzyliden)-indolin-3-on, 2-(4-Dimethylaminobenzyliden)-indolin-3-on, 2-(2-Furylmethylen)-indolin-3-on, 2-(2-Thienylmethylen)-indolin-3-on, 2-(3-Methoxy-4-hydroxybenzyliden)-indolin-3-on, 2-(4-Pyridylmethylen)-indolin-3-on, 5-Brom-2-(4-pyridylmethylen)-indolin-3-on, 2-(2-Pyridylmethylen)-indolin-3-on, 2-(2-Chinolylmethylen)-indolin-3-on, 2-(4-Chinolylmethylen)-indolin-3-on, 2-(3-Oxo-2-indolinylidenmethyl)-1-methylpyridiniumiodid, 4-(1-Methyl-3-oxo-2-indolinylidenmethyl)-1-methylpyridiniumiodid, 2-(1-Methyl-3-oxo-2-indolinylidenmethyl)-1-methylpyridiniumiodid, 4-(3-Oxo-2-indolinylidenmethyl)-1-methylchinoliniummethylsulfonat, 4-(3-Oxo-2-indolinylidenmethyl)-1-methylchinolinium-p-toluolsulfonat, 2-(2-Pyrrolylmethylen)-indolin-3-on, 4-(1-Methyl-3-oxo-2-indolinylidenmethyl)-1-methylpyrroliumiodid sowie deren physiologisch verträglichen Salzen.

Es werden Färbungen mit noch erhöhter Brillanz und verbesserten Echtheitseigenschaften (Lichtechtheit, Waschechtheit, Reibechtheit) über einen weiten Nuancenbereich erzielt. Die Verbindungen der Komponente B sind einerseits Verbindungen, die für sich alleine keratinhaltige Fasern nur schwach färben und erst gemeinsam mit den Verbindungen der Formel I brillante Färbungen ergeben. Andererseits sind darunter aber auch Verbindungen, die bereits als Oxidationsfarbstoffvorprodukte eingesetzt werden.

Die voranstehend genannten Verbindungen mit der Formel I sowie die Verbindungen der Komponente B werden jeweils vorzugsweise in einer Menge von 0,03 bis 65 mmol, insbesondere von 1 bis 40 mmol, bezogen auf 100 g des gesamten Färbemittels, verwendet. Sie können als direktziehende Färbemittel auch in Gegenwart von üblichen Oxidationsfarbstoffvorprodukten und gegebenenfalls Oxidationsmitteln wie z.B. Wasserstoffperoxid eingesetzt werden.

Geeignete Verbindungen mit primärer oder sekundärer Aminogruppe als Komponente B sind z. B. primäre und sekundäre aromatische Amine wie N,N-Dimethyl-p-phenylendiamin, N,N-Diethyl-p-phenylendiamin, N-(2-Hydroxyethyl)-N-ethyl-p-phenylendiamin, N,N-Bis-(2-hydroxyethyl)-p-phenylendiamin, N-(2-Methoxyethyl)-p-phenylendiamin, 2,3-Dichlor-p-phenylendiamin, 2,4-Dichlor-p-phenylendiamin, 2,5-Dichlor-p-phenylendiamin, 2-Chlor-p-phenylendiamin, 2,5-Dihydroxy-4-morpholinoanilin, 2-Aminophenol, 3-Aminophenol, 4-Aminophenol, 2-Aminomethyl-4-aminophenol, 2-Hydroxymethyl-4-aminophenol, o-Phenylendiamin, m-Phenylendiamin, p-Phenylendiamin, 2,5-Diaminotoluol, 2,5,-Diaminophenol, 2,5-Diaminoanisol, 2,5,Diaminophenethol, 4-Amino-3-methylphenol, 2-(2,5-Diaminophenyl)-ethanol, 2,4-Diaminophenoxyethanol, 2-(2,5-Diaminophenoxy)-ethanol, 3-Amino-4-(2-hydroxyethyloxy)phenol, 3,4-Methylendioxyphenol, 3,4-Methylendioxyanilin, 3-Amino-2,4-dichlorphenol, 4-Methylaminophenol, 2-Methyl-5-aminophenol, 3-Methyl-4-aminophenol, 2-Methyl-5-(2-hydroxyethylamino)phenol, 3-Amino-2-chlor-6-methylphenol, 2-Methyl-5-amino-4-chlorphenol, 5-(2-Hydroxyethylamino)-4-methoxy-2-methylphenol, 4-Amino-2-hydroxymethylphenol, 2-(Diethylaminomethyl)-4-aminophenol, 4-Amino-1-hydroxy-2-(2-hydroxyethylaminomethyl)-benzol, 1-Hydroxy-2-amino-5-methyl-benzol, 1-Hydroxy-2-amino-6-methyl-benzol, 2-Amino-5-acetamido-phenol, 1,3-Dimethyl-2,5-diaminobenzol, 5-(3-Hydroxypropylamino-)2-methylphenol, 5-Amino-4-methoxy-2-methylphenol, N,N-Dimethyl-3-aminophenol, N-Cyclopentyl-3-aminophenol, 5-Amino-4-fluor-2-methylphenol, 2,4-Diamino-5-fluortoluol, 2,4-Diamino-5-(2-hydroxyethoxy)-toluol, 2,4-Diamino-5-methylphenetol, 3,5-Diamino-2-methoxy-1-methylbenzol, 2-Amino-4-(2-hydroxyethylamino)-anisol, 2,6-Bis-(2-hydroxyethylamino)-1-methylbenzol, 1,3-Diamino-2,4-dimethoxybenzol, 3,5-Diamino-2-methoxy-toluol, 2-Aminobenzoesäure, 3-Aminobenzoesäure, 4-Aminobenzoesäure, 2-Aminophenylessigsäure, 3-Aminophenylessigsäure, 4-Aminophenylessigsäure, 2,3-Diaminobenzoesäure, 2,4-Diaminobenzoesäure, 2,5-Diaminobenzoesäure, 3,4-Diaminobenzoesäure 3,5-Diaminobenzoesäure, 4-Aminosalicylsäure, 5-Aminosalicylsäure, 3-Amino-4-hydroxybenzoesäure, 4-Amino-3-hydroxy-benzoesäure, 2-Aminobenzolsulfonsäure, 3-Aminobenzolsulfonsäure, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-S-hydroxynaphthalin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 1-(2-Hydroxy-5-aminobenzyl)-2-imidazolidinon, 4-Amino-2-((4-[(5-amino-2-hydroxyphenyl)methyl]-piperazinyl)methyl)phenol, 3,5-Diamino-4-hydroxybrenzcatechin, 1,4-Bis-(4-aminophenyl)-1,4-diazacycloheptan, aromatische Nitrile, wie 2-Amino-4-hydroxybenzonitril, 4-Amino-2-hydroxybenzonitril, 4-Aminobenzonitril, 2,4-Diaminobenzonitril, Nitrogruppen-haltige Aminoverbindungen, wie 3-Amino-6-methylamino-2-nitro-pyridin, Pikraminsäure, [8-[(4-Amino-2-nitrophenyl)-azo]-7-hydroxy-naphth-2-yl]-trimethylammoniumchlorid, [8-((4-Amino-3-nitrophenyl)-azo)-7-hydroxy-naphth-2-yl]-trimethylammoniumchlorid (Basic Brown 17), 1-Hydroxy-2-amino-4,6-dinitrobenzol, 1-Amino-2-nitro-4-[bis-(2-hydroxyethyl)amino]-benzol, 1-Amino-2-[(2-hydroxyethyl)amino]-5-nitrobenzol (HC Yellow Nr. 5), 1-Amino-2-nitro-4-[(2-hydroxyethyl)amino]-benzol (HC Red Nr. 7), 2-Chlor-5-nitro-N-2-hydroxyethyl-1,4-phenylendiamin, 1-[(2-Hydroxyethyl)amino]-2-nitro-4-amino-benzol (HC Red Nr. 3), 4-Amino-3-nitrophenol, 4-Amino-2-nitrophenol, 6-Nitro-o-toluidin, 1-Amino-3-methyl-4-[(2-hydroxyethyl)amino]-6-nitrobenzol (HC Violet Nr. 1), 1-Amino-2-nitro-4-[(2,3-dihydroxypropyl)amino]-5-chlor-benzol (HC Red Nr. 10), 2-(4-Amino-2-nitroanilino)-benzoesäure, 6-Nitro-2,5-diaminopyridin, 2-Amino-6-chlor-4-nitrophenol, 1-Amino-2-(3-nitrophenylazo)-7-phenylazo-8-naphthol-3,6-disulfonsäure Dinatriumsalz (Acid blue Nr. 29), 1-Amino-2-(2-hydroxy-4-nitrophenylazo)-8-naphthol-3,6-disulfonsäure Dinatriumsalz (Palatinchrome green), 1-Amino-2-(3-chlor-2-hydroxy-5-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 1-Amino-5-chlor-4-(2-hydroyethylamino)-2-nitrobenzol, aromatische Aniline bzw. Phenole mit einem weiteren aromatischen Rest, wie sie in der Formel II dargestellt sind 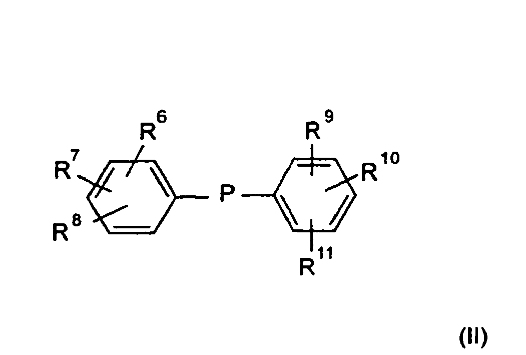 in der
- R⁶ für eine Hydroxy- oder eine Aminogruppe, die durch C₁₋₄-Alkyl-, C₁₋₄-Hydroxyalkyl-, C₁₋₄-Alkoxy- oder C₁₋₄-Alkoxy-C₁₋₄-alkylgruppen substituiert sein kann, steht,
- R⁷, R⁸, R⁹, R¹⁰ und R¹¹ unabhängig voneinander für ein Wasserstoffatom, eine Hydroxy- oder eine Aminogruppe, die durch C₁-C₄-Alkyl-, C₁-C₄-Hydroxyalkyl, C₁-C₄₋Alkoxy-, C₁-C₄-Aminoalkyl- oder C₁-C₄-Alkoxy-C₁-C₄-alkylgruppen substituiert sein kann, stehen, und
- P für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel III

   Q'-(CH₂-Q-CH₂-Q")ₒ (III)
in der
- Q eine direkte Bindung, eine CH₂- oder CHOH-Gruppe bedeutet,
- Q' und Q" unabhängig voneinander für ein Sauerstoffatom, eine NR¹²-Gruppe, worin R¹² ein Wasserstoffatom, eine C₁₋₄-Alkyl- oder eine Hydroxy-C₁₋₄-alkylgruppe, wobei auch beide Gruppen zusammen mit dem Restmolekül einen 5-, 6- oder 7-Ring bilden können, bedeutet, die Gruppe O-(CH₂)ₚ-NH oder NH-(CH₂)_{p'}-O, worin p und p' 2 oder 3 sind, stehen und
- o eine Zahl von 1 bis 4 bedeutet,
wie beispielsweise 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, 4,4'-Diaminodiphenylsulfid, 4,4'-Diaminodiphenylsulfoxid, 4,4'-Diaminodiphenylamin, 4,4'-Diaminodiphenylamin-2-sulfonsäure, 4,4'-Diaminobenzophenon, 4,4'-Diaminodiphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, , 1,3-Bis-(4-aminophenylamino)-2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin und Bis-(5-amino-2-hydroxyphenyl)-methan.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete stickstoffhaltige heterocyclische Verbindungen sind z. B. 2-Aminopyridin, 3-Aminopyridin, 4-Aminopyridin, 2-Amino-3-hydroxy-pyridin, 2,6-Diamino-pyridin, 2,5-Diamino-pyridin, 2-(Aminoethylamino)-5-aminopyridin, 2,3-Diamino-pyridin, 2-Dimethylamino-5-amino-pyridin, 2-Methylamino-3-amino-6-methoxy-pyridin, 2,3-Diamino-6-methoxy-pyridin, 2,6-Dimethoxy-3,5-diamino-pyridin, 2,4,5-Triamino-pyridin, 2,6-Dihydroxy-3,4-dimethylpyridin, N-[2-(2,4-Diaminophenyl)aminoethyl]-N-(5-amino-2-pyridyl)-amin, N-[2-(4-Aminophenyl)aminoethyl]-N-(5-amino-2-pyridyl)-amin, 2,4-Dihydroxy-5,6-diaminopyrimidin, 4,5,6-Triaminopyrimidin, 4-Hydroxy-2,5,6-triaminopyrimidin, 2-Hydroxy-4,5,6-triaminopyrimidin, 2,4,5,6-Tetraaminopyrimidin, 2-Methylamino-4,5,6-triaminopyrimidin, 2,4-Diaminopyrimidin, 4,5-Diaminopyrimidin, 2-Amino-4-methoxy-6-methylpyrimidin, 3,5-Diaminopyrazol, 3,5-Diamino-1,2,4-triazol, 3-Aminopyrazol, 3-Amino-5-hydroxypyrazol, 1-Phenyl-4,5-diaminopyrazol, 1-(2-Hydroxyethyl)-4,5-diaminopyrazol, 1-Phenyl-3-methyl-4,5-diaminopyrazol, 4-Amino-2,3-dimethyl-1-phenyl-3-pyrazolin-5-on (4-Aminoantipyrin), 1-Phenyl-3-methylpyrazol-5-on, 2-Aminochinolin, 3-Aminochinolin, 8-Aminochinolin, 4-Aminochinaldin, 2-Aminonicotinsäure, 6-Aminonicotinsäure, 5-Aminoisochinolin, 5-Aminoindazol, 6-Aminoindazol, 5-Aminobenzimidazol, 7-Aminobenzimidazol, 5-Aminobenzothiazol, 7-Aminobenzothiazol, 2,5-Dihydroxy-4-morpholino-anilin sowie Indol- und Indolinderivaten, wie 4-Aminoindol, 5-Aminoindol, 6-Aminoindol, 7-Aminoindol, 5,6-Dihydroxyindol, 5,6-Dihydroxyindolin und 4-Hydroxyindolin. Weiterhin als heterocyclische Verbindungen können erfindungsgemäß die in der DE-U1-299 08 573 offenbarten Hydroxypyrimidine eingesetzt werden. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Geeignete aromatische Hydroxyverbindungen sind z. B. 2-Methylresorcin, 4-Methylresorcin, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-Methoxyphenol, 3-Methoxyphenol, 4-Methoxyphenol, 3-Dimethylaminophenol, 2-(2-Hydroxyethyl)phenol, 3,4-Methylendioxyphenol, 2,4-Dihydroxybenzoesäure, 3,4-Dihydroxybenzoesäure, 2,4-Dihydroxy-phenylessigsäure, 3,4-Dihydroxy-phenylessigsäure, Gallussäure, 2,4,6-Trihydroxybenzoesäure, 2,4,6-Trihydroxyacetophenon, 2-Chlorresorcin, 4-Chlorresorcin, 1-Naphthol, 1,5-Dihydroxynaphthalin, 2,3-Dihydroxynaphthalin, 2,7-Dihydroxynaphthalin, 6-Dimethylamino-4-hydroxy-2-naphthalinsulfonsäure und 3,6-Dihydroxy-2,7-naphthalinsulfonsäure.

Als Aminosäuren kommen bevorzugt alle natürlich vorkommenden und synthetischen α-Aminosäuren in Frage, z.B. die durch Hydrolyse aus pflanzlichen oder tierischen Proteinen, z.B. Kollagen, Keratin, Casein, Elastin, Sojaprotein, Weizengluten oder Mandelprotein zugänglichen Aminosäuren. Dabei können sowohl sauer als auch alkalisch reagierende Aminosäuren eingesetzt werden. Bevorzugte Aminosäuren sind Arginin, Histidin, Tyrosin, Phenylalanin, DOPA (Dihydroxyphenylalanin), Ornithin, Prolin, Lysin und Tryptophan. Aber auch andere Aminosäuren, wie z.B. 6-Aminocapronsäure und β-Alanin, können eingesetzt werden.

Als CH-acide Verbindungen können beispielhaft genannt werden 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 1,2-Dimethyl-naphtho[1,2-d]thiazolium-p-toluolsulfonat, 1-Ethyl-2-methyl-naphtho[1,2-d]thiazolium-p-toluolsulfonat, Rhodanin, Rhodanin-3-essigsäure, 1-Methyl-2-chinaldinium-iodid, 1-Methyl-2-chinaldinium-p-toluolsulfonat, 1-Ethyl-2-chinaldiniumiodid, 1-Ethyl-2-chinaldinium-p-toluolsulfonat, 1,4-Dimethylchinoliniumiodid, 1,4-Dimethylchinolinium-p-toluolsulfonat, Barbitursäure, Thiobarbitursäure, 1,3-Dimethylthiobarbitursäure, 1,3-Diethylthiobarbitursäure, Oxindol, 3-Indoxylacetat, 2-Cumaranon, 5-Hydroxy-cumaranon, 6-Hydroxy-cumaranon, 1-Methyl-3-phenyl-2-pyrazolinon, Indan-1,3-dion, Indan-1-on, Benzoylacetonitril, 1-Dicyanmethylenindan, 1,3-Bis(dicyanmethylen)indan, 3-Dicyanmethylenindan-1-on, 1,3-Diiminoisoindolin, 2-Amino-4-imino-1,3-thiazolinhydrochlorid, 3-Cyan-1,4-dimethyl-6-hydroxy-2-pyridon und 3-Cyan-1-ethyl-6-hydroxy-4-methyl-2-pyridon.

Die Verbindungen der Komponente B werden besonders bevorzugt ausgewählt aus der Gruppe bestehend aus N-(2-Hydroxyethyl)-N-ethyl-p-phenylendiamin, 2-Chlor-p-phenylendiamin, N,N-Bis-(2-hydroxyethyl)-p-phenylendiamin, 2-Aminophenol, 3-Aminophenol, 4-Aminophenol, 2-Amino-6-chlor-4-nitrophenol, p-Phenylendiamin, 2-(2,5-Diaminophenyl)-ethanol, 2,5-Diaminotoluol, 3,4-Methylendioxyanilin, 2-Amino-4-(2-hydroxyethylamino)-anisol, 2-(2,4-Diaminophenoxy)-ethanol, 3-Amino-2,4-dichlorphenol, 2-Methyl-5-aminophenol, 3-Methyl-4-aminophenol, 2-Methyl-5-(2-hydroxyethylamino)phenol, 2-Methyl-5-amino-4-chlorphenol, 6-Methyl-3-amino-2-chlorphenol, 2-Aminomethyl-4-aminophenol, 2-Diethylaminomethyl-4-aminophenol, 2-Dimethylaminomethyl-4-aminophenol, 2,6-Dichlor-4-aminophenol, 2-Hydroxymethyl-4-aminophenol, 2,6-Bis(2-hydroxyethylamino)-1-methylbenzol, Bis-(2-hydroxy-5-aminophenyl)methan, Bis-(4,5-amino-2-hydroxyphenyl)-methan, 1,3-Bis(2,4-diaminophenoxy)propan, 1,4-Bis(4-aminophenyl)-1,4-diazacycloheptan, 1,8-Bis(2,5-Diaminophenoxy)-3,6-dioxaoctan, 4,4'-Diaminodiphenylamin, 3,4-Methylendioxyphenol, 3,4-Diaminobenzoesäure, 2,5-Diaminopyridin, 2-Dimethylamino-5-aminopyridin, 2-Amino-3-hydroxypyridin, 3-Amino-2-methylamino-6-methoxypyridin, 2,3-Diamino-6-methoxypyridin, 3,5-Diamino-2,6-dimethoxypyridin, 2,6-Dihydroxy-3,4-dimethylpyridin, 2-Hydroxy-4,5,6-triaminopyrimidin, 4-Hydroxy-2,5,6-triaminopyrimidin, 2,4,5,6-Tetraaminopyrimidin, 2-Methylamino-4,5,6-triamino-pyrimidin, 3,5-Diaminopyrazol, 3-Amino-5-hydroxypyrazol, 4,5-Diamino-1-(2-hydroxyethyl)-pyrazol, 5,6-Dihydroxyindol, 5,6-Dihydroxyindolin, 4-Amino-2,3-dimethyl-1-phenyl-3-pyrazolin-5-on (4-Aminoantipyrin), 2,3-Dimethylbenzothiazolium-p-toluolsulfonat, 1,2,3,3-Tetramethyl-3H-indolinium-p-toluolsulfonat, Thiobarbitursäure, Rhodanin, 2,3-Dimethyl-benzothiazolium-p-toluolsulfonat, 1,2-Dimethyl-naphtho[1,2-d]thiazolium-p-toluolsulfonat, 1-Methyl-2-chinaldinium-p-toluolsulfonat, 1,4-Dimethylchinolinium-p-toluolsulfonat, β-Alanin, L-Prolin, L-Lysin, DL-Tyrosin sowie deren vorzugsweise mit anorganischen Säuren gebildeten physiologisch verträglichen Salze.

Ganz besonders bevorzugte Verbindungen der Komponente B sind erfindungsgemäß N,N-Bis(2-hydroxyethyl)-p-phenylendiamin, 2,5-Diaminotoluol, 2-(2,5-Diaminophenyl)-ethanol, 2-Aminophenol, 3-Aminophenol, 4-Aminophenol, 2-Aminomethyl-4-aminophenol, 2-(Diethylaminomethyl)-4-aminophenol, 1-Hydroxy-2-amino-5-methylbenzol, 2,4-Diaminophenoxyethanol, 3-Amino-2,4-dichlorphenol, 2-Methyl-5-aminophenol, 2-Methyl-5-(2-hydroxyethylamino)-phenol, 6-Methyl-3-amino-2-chlorphenol, 2-Methyl-5-amino-4-chlorphenol, 3,4-Methylendioxyanilin, 3,4-Diaminobenzoesäure, 3,5-Diamino-2-methoxy-1-methylbenzol, 2-Amino-4-(2-hydroxyethylamino)anisol, 2,6-Bis-(2-hydroxyethylamino)-1-methylbenzol, 1,3-Bis-(2,4-diaminophenoxy)propan, 4,5-Diamino-1-(2-hydroxyethyl)pyrazol, 1,4-Bis(4-aminophenyl)-1,4-diazacycloheptan, Bis-(2-hydroxy-5-aminophenyl)methan, 1,8-Bis-(2,5-diaminophenoxy)-3,6-dioxaoctan, 4,4'-Diaminodiphenylamin, 2-Amino-3-hydroxypyridin, 3-Amino-2-methylamino-6-methoxypyridin, 2,6-Dihydroxy-3,4-dimethylpyridin, 5,6-Dihydroxyindol, 5,6-Dihydroxyindolin, 2,4,5,6-Tetraaminopyrimidin, 2-Hydroxy-4,5,6-triamino-pyrimidin, 4-Hydroxy-2,5,6-triamino-pyrimidin sowie deren mit vorzugsweise anorganischen Säuren gebildeten physiologisch verträglichen Salze.

Zur Erlangung weiterer und intensiverer Ausfärbungen können die erfindungsgemäßen Mittel zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Arginin, Histidin, Pyrrolidin, Prolin, Pyrrolidon, Pyrrolidon-5-carbonsäure, Pyrazol, 1,2,4-Triazol, Piperazidin, deren Derivate sowie deren physiologisch verträglichen Salzen.

Die voranstehend genannten Farbverstärker können in einer Menge von jeweils 0,03 bis 65 mmol, insbesondere 1 bis 40 mmol, jeweils bezogen auf 100 g des gesamten Färbemittels, eingesetzt werden.

Auf die Anwesenheit von Oxidationsmitteln, z. B. H₂O₂, kann dabei verzichtet werden. Es kann jedoch u. U. wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂. Auch Gemische von mehreren Oxidationsmitteln, wie beispielsweise eine Kombination aus Wasserstoffperoxid und Peroxodisulfaten der Alkail- und Erdalkalimetalle oder aus lodidionenquellen, wie z.B. Alkalimetalliodiden und Wasserstoffperoxid oder den vorgenannten Peroxodisulfaten, können verwendet werden. Das Oxidationsmittel bzw. die Oxidationsmittelkombination können erfindungsgemäß in Verbindung mit Oxidationskatalysatoren in dem Haarfärbemittel zur Anwendung kommen. Oxidationskatalysatoren sind beispielsweise Metallsalze oder Metalloxide, die einen leichten Wechsel zwischen zwei Oxidationsstufen der Metallionen ermöglichen. Beispiele sind Salze oder Oxide von Eisen, Ruthenium, Mangan und Kupfer.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, z. B. aus der Gruppe der Nitrophenylendiamine, Nitroaminophenole, Anthrachinone oder Indophenole, wie z. B. die unter den internationalen Bezeichnungen bzw. Handelsnamen HC Yellow 2, HC Yellow 4, HC Yellow 6, Basic Yellow 57, Disperse Orange 3, HC Red BN, Basic Red 76, HC Blue 2, Disperse Blue 3, Basic Blue 99, Disperse Violet 1, Disperse Violet 4, Disperse Black 9, Basic Brown 16 und Basic Brown 17 bekannten Verbindungen sowie 6-Nitro-1,2,3,4-tetrahydrochinoxalin, 4-N-Ethyl-1,4-bis-(2-hydroxyethylamino)-2-nitrobenzolhydrochlorid und 1-Methyl-3-nitro-4-(2-hydroxyethyl)-aminobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, enthalten.

Es ist nicht erforderlich, daß die Oxidationsfarbstoffvorprodukte oder die fakultativ enthaltenen direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z. B. Cremes, Emulsionen, Gele oder auch tensidhaltige schäumende Lösungen wie z. B. Shampoos oder andere Zubereitungen, die für die Anwendung auf den keratinhaltigen Fasern geeignet sind. Falls erforderlich ist es auch möglich, die Färbemittel in wasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓSO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Akyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈₋Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quatemisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Coming 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quatemierte Dimethylaminoethylmethacrylat-Vinylpyrrolidon-Copolymere, Vinylpyrrolidon-Imidazoliniummethochlorid-Copolymere und quatemierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.-Butylaminoethylmethacrylat/2-Hydroxypropylmethacrylat-Copolymere.
- anionische Polymere wie beispielsweise Polyacrylsäuren, vemetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethytacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind z. B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65 mmol, insbesondere von 1 bis 40 mmol, bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 10.

Ein zweiter Gegenstand der vorliegenden Erfindung betrifft die Verwendung von 2-Arylidenindolin-3-on-Derivaten gemäß Formel I und/oder deren physiologisch verträglichen Salzen, 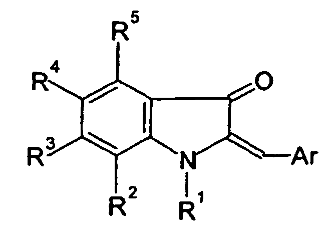 wobei R¹, R², R³, R⁴, R⁵ und Ar wie oben definiert sind, in Kombination mit mindestens einer Verbindung, ausgewählt aus (a) Verbindungen mit primärer oder sekundärer Amino- oder Hydroxygruppe, ausgewählt aus aromatischen Hydroxyverbindungen, primären oder sekundären aromatischen Aminen und stickstoffhaltigen heterozyklischen Verbindungen, (b) Aminosäuren und (c) CH-aciden Verbindungen, als färbende Komponente in Haarfärbemitteln.

Ein dritter Gegenstand der vorliegenden Erfindung betrifft ein Verfahren zum Färben von keratinhaltigen Fasern, insbesondere menschlichen Haaren, worin ein Färbemittel, enthaltend mindestens ein 2-Arylidenindolin-3-on-Derivat gemäß Formel I und/oder deren physiologisch verträglichen Salzen, 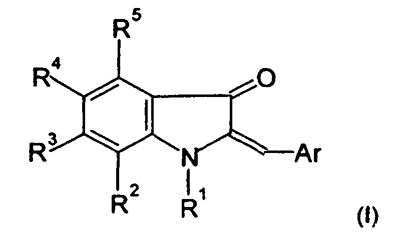 wobei R¹, R², R³, R⁴, R⁵ und Ar wie oben definiert sind, in Kombination mit mindestens einer Verbindung, ausgewählt aus (a) Verbindungen mit primärer oder sekundärer Amino- oder Hydroxygruppe, ausgewählt aus aromatischen Hydroxyverbindungen, primären oder sekundären aromatischen Aminen und stickstoffhaltigen heterozyklischen Verbindungen, (b) Aminosäuren und (c) CH-aciden Verbindungen, sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

Dabei können die 2-Arylidenindolin-3-on-Derivate gemäß Formel I und die Verbindungen der Komponente B als farbgebende Komponenten entweder gleichzeitig auf das Haar aufgebracht werden oder aber auch nacheinander, d. h. in einem mehrstufigen Verfahren, wobei es unerheblich ist, welche der Komponenten zuerst aufgetragen wird. Die fakultativ enthaltenen Ammonium- oder Metallsalze können dabei den Verbindungen mit der Formel I oder den Verbindungen der Komponente B zugesetzt werden. Zwischen dem Auftragen der einzelnen Komponenten können bis zu 30 Minuten Zeitabstand liegen. Auch eine Vorbehandlung der Fasern mit der Salzlösung ist möglich.

Enthält das Haarfärbemittel neben den Verbindungen gemäß Formel I und der Komponente B zusätzlich als Oxidationsmittel Wasserstoffperoxid oder ein wasserstoffperoxidhaltiges Oxidationsmittelgemisch, so liegt der pH-Wert des wasserstoffperoxidhaltigen Haarfärbemittels vorzugsweise in einem pH-Bereich von pH 7 bis pH 11, besonders bevorzugt pH 8 bis pH 10. Das Oxidationmittel kann unmittelbar vor der Anwendung mit dem Haarfärbemittel gemischt und die Mischung auf das Haar aufgebracht werden. Werden die Verbindungen der Formel I und die Komponente B in einem zweistufigen Verfahren auf das Haar appliziert, ist das Oxidationsmittel in einer der beiden Verfahrensstufen zusammen mit der entsprechenden farbgebenden Komponente anzuwenden. Zu diesem Zweck kann es bevorzugt sein, das Oxidationsmittel mit einer der farbgebenden Komponenten in einem Container zu konfektionieren.

Die 2-Arylidenindolin-3-on-Derivate gemäß Formel I und die Verbindungen der Komponente B können entweder getrennt oder zusammen gelagert werden, entweder in einer flüssigen bis pastösen Zubereitung (wässrig oder wasserfrei) oder als trockenes Pulver. Werden die Komponenten in einer flüssigen Zubereitung zusammen gelagert, so sollte diese zur Verminderung einer Reaktion der Komponenten weitgehend wasserfrei sein. Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen (30°C bis 80°C) Wassers hinzugefügt und eine homogene Mischung hergestellt.

### Beispiels

### Herstellung einer Färbelösung

Es wurde je eine Aufschlämmung bzw. Lösung von 5 mmol des 2-Arylidenindolin-3-on-Derivats der Formel I (Komponente A) sowie 5 mmol der Verbindungen der Komponente B mit 5 mmol Natriumacetat in 50 ml Wasser bei ca. 50°C hergestellt. Die Aufschlämmungen bzw. Lösungen wurden nach Abkühlen auf 30°C miteinander vermischt und der pH-Wert auf pH 6 mit einer verdünnten wäßrigen NaOH-Lösung eingestellt.

### Ausfärbungen

In die frisch hergestellte Färbelösung wurde bei 30°C 30 Minuten lang eine Strähne zu 90% ergrauten, nicht vorbehandelten Menschenhaares eingebracht. Die Strähne wurde dann 30 Sekunden mit lauwarmem Wasser gespült, mit warmer Luft (30°C bis 40°C) getrocknet und anschließend ausgekämmt.

Die jeweiligen Farbnuancen und Farbtiefen der Beispielausfärbungen sind in der nachfolgenden Tabelle 1 wiedergegeben.

Die Farbtiefe wurde dabei nach folgender Skala bewertet:

| | | |
|---|---|---|
| - | : | keine oder eine sehr blasse Ausfärbung |
| (+) | : | schwache Intensität |
| + | : | mittlere Intensität |
| +(+) | : | mittlere bis starke Intensität |
| ++ | : | starke Intensität |
| ++(+) | : | starke bis sehr starke Intensität |
| +++ | : | sehr starke Intensität |

### Verbindungen der Komponente A (Tabelle 1):

| | |
|---|---|
| A1 | 4-(3-Oxo-2-indolinylidenmethyl)-1-methylpyridiniumiodid |
| A2 | 2-(4-Pyridylmethylen)indolin-3-on |
| A3 | 2-(2-Pyrrolylmethylen)indolin-3-on |

### Verbindungen der Komponente B (Tabelle 1):

| | |
|---|---|
| B1 | 2,5-Diaminotoluol H₂SO₄ |
| B2 | 3-Amino-2-methylamino-6-methoxypyridin |
| B3 | 2-Aminomethyl-4-aminophenol 2 HCI |
| B4 | N,N-Bis(2-hydroxyethyl)-p-phenylendiamin H₂SO₄ |
| B5 | 3,5-Diamino-2,6-dimethoxypyridin 2 HCI |
| B6 | 2-Amino-4-(2-hydroxyethylamino)anisol (Lehmanns Blau) |

**Tabelle 1**

| **Komponente A** | **Komponente B** | **Farbe** | **Farbtiefe** |
|---|---|---|---|
| A1 | - | leuchtend rot | ++ |
| A1 | B1 | violettrot | ++(+) |
| A1 | B2 | schwarzbraun | +++ |
| A1 | B3 | rotviolett | ++ |
| A1 | B4 | rotviolett | ++(+) |
| A1 | B5 | schwarz | +++ |
| A2 | - | hellorange | (+) |
| A2 | B2 | gelbbraun | ++ |
| A2 | B4 | violettbraun | +(+) |
| A3 | - | rosarot | + |
| A3 | B1 | rotviolett | +(+) |
| A3 | B2 | dunkelbraun | ++(+) |
| A3 | B4 | rotviolett | ++ |
| A3 | B5 | braunschwarz | +++ |
| A3 | B6 | rotviolett | ++ |

## Patentansprüche

1. Mittel zum Färben von keratinhaltigen Fasern, Insbesondere menschlichen Haaren, enthaltend mindestens ein 2-Arylidenindolin-3-on-Derivat gemäß Formel I und/oder deren physiologisch verträglichen Salze, 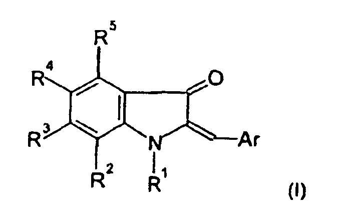 wobei
• R¹ steht für ein Wasserstoffatom, eine C₁-C₄-Alkylgruppe, eine C₂-C₆-Alkenylgruppe, eine Arylgruppe, eine Aryl-C₁-C₄-alkylgruppe, eine C₁-C₄-Acylgruppe, eine C₁-C₄-Hydroxyalkylgruppe, eine C₂-C₄-Dihydroxyalkylgruppe, eine C₁-C₄-Sulfoalkylgruppe, eine C₁-C₄-Carboxyalkylgruppe, eine Gruppe R^{I}R^{II}N-(CH₂)ₘ-, worin R^{I} und R^{II} stehen unabhängig voneinander für ein Wasserstoffatom, eine C₁-C₄-Alkylgruppe, eine C₁-C₄-Hydroxyalkylgruppe oder eine Aryl-C₁-C₄-alkylgruppe, wobei R^{I} und R^{II} gemeinsam mit dem Stickstoffatom einen 5-. 6- oder 7-gliedrigen Ring bilden können und m steht für eine Zahl 1, 2, 3 oder 4,
• R², R³, R⁴ und R⁵ stehen unabhängig voneinander für ein Wasserstoffatom, ein Halogenatom, eine C₁-C₄₋Alkylgruppe, eine C₁-C₄-Alkoxygruppe, eine C₁-C₄-Hydroxyalkoxygruppe, eine Hydroxygruppe, eine Nitrogruppe, eine Carboxygruppe, eine Sulfonsäuregruppe, eine Sulfamoylgruppe, eine Gruppe R^{III}R^{IV}N-(CH₂)_{q}-. worin R^{III} und R^{IV} stehen unabhängig voneinander für ein Wasserstoffatom, eine C₁-C₄-Alkylgruppe, eine C₁-C₄-Hydroxyalkylgruppe oder eine Aryl-C₁-C₄-alkylgruppe und q ist steht für eine Zahl 0, 1, 2, 3 oder 4, wobei auch zwei der Reste R², R³, R⁴ und R⁵ zusammen mit dem Benzolring des 2-Arylidenindolin-3-on-Grundkörpers einen 5- oder 6-gliedrigen, carbozyktischen oder heterozyklischen, aliphatischen oder aromatischen Ring bilden können, und
• Ar steht für Phenyl, Naphthyl, Styryl, Pyridyl, Pyrimidyl, Pyrazyl, Pyrazidyl, Pyrrolyl, Furyl, Thienyl, 1,2,3-Triazinyl, 1,3,5-Triazinyl, Chinolyl, Isochinolyl, Indolyl, Indolinyl, 3-Oxo-indolinyl, Indolizinyl, Indanyl, Imidazolyl, 1,2,4-Triazolyl, 1,2,3-Triazolyl, Tetrazolyl, Benzimidazolyl, 1,3-Thiazolyl, Benzothiazolyl, Indazolyl, Benzoxazolyl, Chinoxalinyl, Chinazolinyl und Cinnolinyl, wobei der Ar-Rest gegebenenfalls durch ein Halogenatom, eine C₁-C₄-Alkylgruppe, eine C₁-C₄-Alkoxygruppe, eine C₁-C₄-Hydroxyalkylgruppe, eine Hydroxygruppe, eine Carboxygruppe, eine Nitrogruppe, eine Sulfonsäuregruppe oder eine Gruppe R^{V}R^{VI}N-(CH₂)ᵣ-, worin R^{V} und R^{VI} stehen unabhängig voneinander für ein Wasserstoffatom, eine C₁-C₄-Alkylgruppe, eine C₁-C₄-Hydroxyalkylgruppe oder eine Aryl-C₁-C₄-alkylgruppe und r steht für eine Zahl 0, 1, 2, 3 oder 4, substituiert ist
und zusätzlich als Komponente B mindestens eine Verbindung, ausgewählt aus (a) Verbindungen mit primärer oder sekundärer Amino- oder Hydroxygruppe, ausgewählt aus aromatischen Hydroxyverbindungen, primären oder sekundären aromatischen Aminen und stickstoffhaltigen heterozyklischen Verbindungen, (b) Aminosäuren und (c) CH-aciden Verbindungen.

2. Mittel nach Anspruch 1, **dadurch gekennzeichnet, daß** die 2-Arylidenindolin-3-on-Derivate der Formel ausgewählt sind aus 2-Benzylidenindolin-3-on, 2-(2-Hydroxybenzyliden)indofin-3-on, 2-(4-Hydroxybenzyliden)indolin-3-on, 2-(4-Methoxybenzyliden)indolin-3-on, 2-(4-Methoxybenzyliden)-5-nitroindolin-3-on, 2-(4-Nitrobenzyliden)-4-nitro-indolin-3-on, 2-(4-Nitrobenzyliden)-5-methoxyindolin-3-on, 2-(3-Nitrobenzyliden)indolin-3-on, 2-(2-Nitrobenzyliden)indolin-3-on, 1-Methyl-2-(4-nitrobenzyliden)indolin-3-on, 1-Methyl-2-(3-Nitrobenzyliden)indolin-3-on, 1-Methyl-2-(2-nitrobenzyliden)indolin-3-on, 2-(3,4-Dimethoxybenzyliden)indolin-3-on, 2-(3,4-Methylendioxybenzyliden)indolin-3-on, 2-(4-Dimethylaminobenzyliden)indolin-3-on, 2-(2-Furylmethylen)indolin-3-on, 2-(2-Thienylmethylen)indolin-3-on, 2-(3-Methoxy-4-hydroxybenzyliden)indolin-3-on, 2-(4-Pyridylmethylen)indolin-3-on,5-Brom-2-(4-pyridylmethylen)indolin-3-on,2-(2-Pyridylmethylen)indolin-3-on,2-(2-Chinotylmethylen)indolin-3-on, 2-(4-Chinolylmethylen)indolin-3-on, 2-(3-Oxo-2-indolinylidenmelhyl)-1-melhylpyridiniumiodid, 4-(1-Methyl-3oxo-2-indolinylidenmethyl)-1-methylpyridiniumiodid, 2-(1-Methyl-3-oxo-2-indolinylidenmethyl)-1-methylpyridiniumiodid, 4-(3-Oxo-2-Indolinylidenmethyl)-1-methylchinoliniummethylsulfonat, 4-(3-Oxo-2-indolinylidenmethyl)-1-methylchinolinium-p-toluolsulfonat, 2-(2-Pyrrolylmethyten)-indolin-3-on, 4-(1-Methyl-3-oxo-2-indolinylidenmethyl)-1-methylpyrroliumiodid sowie deren physiologisch verträglichen Salzen.

3. Mittel nach einem der Ansprüche 1 oder 2, **dadurch gekennzeichnet, daß** die Verbindungen der Formel und die Verbindungen der Komponente B jeweils in einer Menge von 0,03 bis 65 mmol, insbesondere von 1 bis 40 mmol, bezogen auf 100 g des gesamten Färbemittels, enthalten sind.

4. Mittel nach einem der Ansprüche 1 bis 3, **dadurch gekennzeichnet, daß** die Verbindung der Komponente B ausgewählt ist aus N,N-Dimethyl-p-phenylendiamin, N,N-Diethyl-p-phenylendiamin, N-(2-Hydroxyethyl)-N-ethyl-p-phenylendiamin, N,N-Bis-(2-hydroxyethyl)-p-phenylendiamin, N-(2-Methoxyethyl)-p-phenylendiamin, 2,3-Dichlor-p-phenylendiamin, 2,4-Dichlor-p-phenylendiamin, 2,5-Dichlor-p-phenylendiamin, 2-Chlor-p-phenylendiamin, 2,5-Dihydroxy-4-morpholinoanilin, 2-Aminophenol, 3-Aminophenol, 4-Aminophenol, 2-Aminomethyl-4-aminophenol, 2-Hydroxymethyl-4-aminophenol, o-Phenylendiamin, m-Phenylendiamin, p-Phenylendiamin, 2,5-Diaminotoluol, 2,5,-Diaminophenol, 2,5-Diaminoanisol, 2,5,Diaminophenethol, 4-Amino-3-methylphenol, 2-(2,5-Diaminophenyl)-ethanol, 2,4-Diaminophenoxyethanol, 2-(2,5-Diaminophenoxy)-ethanol, 3-Amino-4-(2-hydroxyethyloxy)phenol, 3,4-Methylendioxyphenol, 3,4-Methylendioxyanilin, 3-Amino-2,4-dichlorphenol, 4-Methylaminophenol, 2-Methyl-5-aminophenol, 3-Methyl-4-aminophenol, 2-Methyl-5-(2-hydroxyethylamino)phenol, 3-Amino-2-chlor-6-methylphenol, 2-Methyl-5-amino-4-chlorphenol, 5-(2-Hydroxyethylamino)-4-methoxy-2-methylphenol, 4-Amino-2-hydroxymethylphenol, 2-(Diethylaminomethyl)-4-aminophenol, 4-Amino-1-hydroxy-2-(2-hydroxyethylaminomethyl)-benzol, 1-Hydroxy-2-amino-5-methyl-benzol, 1-Hydroxy-2-amino-6-methyl-benzol, 2-Amino-5-acetamido-phenol, 1,3-Dimethyl-2,5-diaminobenzol, 5-(3-Hydroxypropylamino-)2-methylphenol, 5-Amino-4-methoxy-2-methylphenol, N,N-Dimethyl-3-aminophenol, N-Cyclopentyl-3-aminophenol, 5-Amino-4-fluor-2-methylphenol, 2,4-Diamino-5-fluortoluol, 2,4-Diamino-5-(2-hydroxyethoxy)-toluol, 2,4-Diamino-5-methylphenetol, 3,5-Diamino-2-methoxy-1-methylbenzol, 2-Amino-4-(2-hydroxyethylamino)-anisol, 2,6-Bis-(2-hydroxyethylamino)-1-methylbenzol, 1,3-Diamino-2,4-dimethoxybenzol, 3,5-Diamino-2-methoxy-toluol, 2-Aminobenzoesäure, 3-Aminobenzoesäure, 4-Aminobenzoesäure, 2-Aminophenylessigsäure, 3-Aminophenylessigsäure, 4-Aminophenylessigsäure, 2,3-Diaminobenzoesäure, 2,4-Diaminobenzoesäure, 2,5-Diaminobenzoesäure, 3,4-Diaminobenzoesäure 3,5-Diaminobenzoesäure, 4-Aminosalicylsäure, 5-Aminosalicylsäure, 3-Amino-4-hydroxy-benzoesäure, 4-Amino-3-hydroxybenzoesäure, 2-Aminobenzolsulfonsäure, 3-Aminobenzolsulfonsäure, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 1-(2-Hydroxy-5-aminobenzyl)-2-imidazolidinon, 4-Amino-2-((4-[(5-amino-2-hydroxyphenyl)methyl]-piperazinyl)methyl)phenol, 3,5-Diamino-4-hydroxybrenzcatechin, 1,4-Bis-(4-aminophenyl)-1,4-diazacycloheptan, aromatische Nitrile, wie 2-Amino-4-hydroxybenzonitril, 4-Amino-2-hydroxybenzonitril, 4-Aminobenzonitril, 2,4-Diaminobenzonitril, Nitrogruppen-haltige Aminoverbindungen, wie 3-Amino-6-melhylamino-2-nitro-pyridin, Pikraminsäure, [8-[(4-Amino-2-nitrophenyl)-azo]-7-hydroxy-naphth-2-yl}-trimethylammoniumchlorid, [8-((4-Amino-3-nitrophenyl)-azo)-7-hydroxy-naphth-2-yl]-trimethylammoniumchlorid (Basic Brown 17), 1-Hydroxy-2-amino-4,6-dinitrobenzol, 1-Amino-2-nitro-4-[bis-(2-hydroxyethl)amino]-benzol, 1-Amina2-[(2-hydoxyethyl)amino]-5-nitrobenzol (HC Yellow Nr. 5), 1-Amino-2-nitro-4-[(2-hydroxyethyl)amino]-benzol (HC Red Nr. 7), 2-Chlor-5-nitro-N-2-hydroxyethyl-1,4-phenylendiamin, 1-[(2-Hydroxyethyl)amino]-2-nitro-4-amino-benzol (HC Red Nr. 3), 4-Amino-3-nitrophenol, 4-Amino-2-nitrophenol, 6-Nitro-o-toluidin, 1-Amino-3-methyl-4-((2-hydroxyethyl)amino)-6-nitrobenzol (HC Violet Nr. 1), 1-Amino-2-nitro-4-[(2,3-dihydroxypropyl)amino]-5-chlor-benzol (HC Red Nr. 10), 2-(4-Amino-2-nitroanilino)-benzoesäure, 6-Nitro-2,5-diaminopyridin, 2-Amino-6-chlor-4-nitrophenol, 1-Amino-2-(3-nitrophenylazo)-7-phenylazo-8-naphthol-3,6-disulfonsäure Dinatriumsalz (Add blue Nr. 29), 1-Amino-2-(2-hydroxy-4-nitrophenylazo)-8-naphthol-3,6-disulfonsäure Dinatriumsalz (Palatinchrome green), 1-Amino-2-(3-chlor-2-hydroxy-5-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'dinitro-2'-hydroxy-5-methylazobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)ethylamino]-5-nitroanilin, 3-Amino-4-hydroxy-5-nitrobenzolsulfonsaure 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin. 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin,2-Nitro-4-amino-1-(2-hydroxyethylamino)-benzol,2-Nitro-1-amino-4-[bis-(2-hydroxyethyl)-amino]-benzol, 4-Amino-2-nitrodiphenylamin-2'-carbonsäure, 1-Aminas-chlor-4-(2-hydroyethylamino)-2-ni trobenzol, 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, 4,4'-Diaminodiphenylsulfid, 4,4'-Diaminodiphenylsulfoxid, 4,4'-Diaminodiphenylamin, 4,4'-Diaminodiphenylamin-2-sulfonsäure, 4,4'-Diaminobenzophenon, 4,4'-Diaminodiphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, 1,3-Bis-(4-aminophenylamino)-2-propanol, 1,3-Bis-[N-(4-aminophenyl)-2-hydroxyethylamino]-2-propanol, N,N-Bis-[2-(4-aminophenoxy)ethyl]methylamin, N-Phenyl-1,4-phenylendiamin und Bis-(5-amino-2-hydroxyphenyl)-methan, stickstoffhaltigen heterocyclischen Verbindungen ausgewählt aus der Gruppe bestehend aus 2-Aminopyridin, 3-Aminopyridin, 4-Aminopyridin, 2-Amino-3-hydroxypyridin, 2,6-Diamino-pyridin, 2,5-Diamino-pyridin, 2-(Aminoethylamino)-5-aminopyridin, 2,3-Diamino-pyridin, 2-Dimethylamino-5-amino-pyridin, 2-Methylamino-3-amino-6-methoxy-pyridin, 2,3-Diamino-6-methoxy-pyridin, 2,6-Dimethoxy-3,5-diamino-pyridin, 2,4,5-Triamino-pyridin, 2,6-Dihydroxy-3,4-dimethylpyridin, N-[2-(2,4-Diaminophenyl) aminoethyl]-N-(5-amino-2-pyridyl)-amin, N-[2-(4-Aminophenyl)aminoethyl]-N-(5-amino-2-pyridyl)-amin, 2,4-Dihydroxy-5,6-diaminopyrimidin, 4,5,6-Triaminopyrimidin, 4-Hydroxy-2,5,6-triaminopyrimidin, 2-Hydroxy-4,5,6-triaminopyrimidin, 2,4,5,6-Tetraaminopyrimidin, 2-Methylamino-4,5,6-triaminopyrimidin, 2,4-Diaminopyrimidin, 4,5-Diaminopyrimidin, 2-Amino-4-methoxy-6-methyl-pyrimidin, 3,5-Diaminopyrazol, 3,5-Diamino-1,2,4-triazol, 3-Aminopyrazol, 3-Amino-5-hydroxypyrazol, 1-Phenyl-4,5-diaminopyrazol, 1-(2-Hydroxyethyl)-4,5-diaminopyrazol, 1-Phenyl-3-methyl-4,5-diaminopyrazol, 4-Amino-2,3-dimethyl-1-phenyl-3-pyrazolin-5-on (4-Aminoantipyrin), 1-Phenyl-3-methylpyrazol-5-on, 2-Aminochinolin, 3-Aminochinolin, 8-Aminochinolin, 4-Aminochinaldin, 2-Aminonicotinsäure, 6-Aminonicotinsäure, 5-Aminoisochinolin, 5-Aminoindazol, 6-Aminoindazol, 5-Aminobenzimidazol, 7-Aminobenzimidazol, 5-Aminobenzothiazol, 7-Aminobenzothiazol, 2,5-Dihydroxy-4-morpholino-anilin sowie Indol-und Indolinderivaten, wie 4-Aminoindol, 5-Aminoindol, 6-Aminoindol, 7-Aminoindol, 5,6-Dihydroxyindol, 5,6-Dihydroxyindolin, 4-Hydroxyindolin, Hydroxypyrimidin-Derivate, sowie aromatischen Hydroxyverbindungen wie 2-Methylresorcin, 4-Methylresorcin, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-Methoxyphenol, 3-Methoxyphenol, 4-Methoxyphenol, 3-Dimethylaminophenol, 2-(2-Hydroxyethyl)phenol, 3,4-Methylendioxyphenol, 2,4-Dihydroxybenzoesäure, 3,4-Dihydroxybenzoesäure, 2,4-Dihydroxy-phenylessigsäure, 3,4-Dihydroxy-phenylessigsäure, Gallussäure, 2,4,6-Trihydroxybenzoesäure, 2,4,6-Trihydroxyacetophenon, 2-Chlorresorcin, 4-Chlorresorcin, 1-Naphthol, 1,5-Dihydroxynaphthalin, 2,3-Dihydroxynaphthalin, 2,7-Dihydroxynaphthalin, 6-Dimethylamino-4-hydroxy-2-naphthalinsulfonsäure und 3,6-Dihydroxy-2,7-naphthalinsulfonsäure, Aminosäuren ausgewählt aus der Gruppe Arginin, Histidin, Tyrosin, Phenylalanin, DOPA (Dihydroxyphenylalanin), Omithin, Prolin, Lysin, Tryptophan, 6-Aminocapronsäure und β-Alanin, CH-aciden Verbindungen ausgewählt aus der Gruppe 1,2,3,3-Tetramethyl-3H-indoliumiodid, 1,2,3,3-Tetramethyl-3H-indolium-p-toluolsulfonat, 5,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimelhyl-benzothiazolium-p-toluolsutfonat, 1,2-Dimethyl-naphtho[1,2-d]thiazolium-p-toluolsulfonat, 1-Ethyl-2-methylnaphtho [1,2-d]thiazolium-p-toluolsulfonat, Rhodanin, Rhodanin-3-essigsäure, 1-Methyl-2-chinaldinium-iodid, 1-Methyl-2-chinaldinium-p-toluolsulfonat, 1-Ethyl-2-chinaldiniumiodid, 1-Ethyl-2-chinaldinium-p-toluolsulfonat, 1,4-Dimethylchinoliniumiodid, 1,4-Dimethylchinolinium-p-toluolsutfonat, Barbitursäure, Thiobarbitursäure, 1,3-Dimethylthiobarbitursäure, 1,3-Diethylthiobarbitursäure, Oxindol, 3-Indoxylacetat, 2-Cumaranon, 5-Hydroxy-cumaranon, 6-Hydroxy-cumaranon, 1-Methyl-3-phenyl-2-pyrazolinon, Indan-1,3-dion, Indan-1-on, Benzoylacetonitril, 1-Dicyanmethylenindan, 1,3-Bis(dicyanmethylen)indan, 3-Dicyanmethylenindan-1-on, 1,3-Diiminoisoindolin, 2-Amino-4-imino-1,3-thiazolinhydrochlorid, 3-Cyan-1,4-dimethyl-6-hydroxy-2-pyridon und 3-Cyan-1-ethyl-6-hydroxy-4-methyl-2-pyridon, sowie den physiologisch verträglichen Salzen aller vorgenannten Verbindungen.

5. Mittel nach einem der Ansprüche 1 bis 4, **dadurch gekennzeichnet, daß** die Komponente B ausgewählt ist aus Verbindungen der Gruppe bestehend aus N-(2-Hydroxyethyl)-N-ethyl-p-phenylendiamin, 2-Chlor-p-phenylendiamin, N,N-Bis-(2-hydroxyethyl)-p-phenylendiamin, 2-Aminophenol, 3-Aminophenol, 4-Aminophenol, 2-Amino-6-chlor-4-nitrophenol, p-Phenylendiamin, 2-(2,5-Diaminophenyl)-ethanol, 2,5-Diaminotoluol, 3,4-Methylendioxyanilin, 2-Amino-4-(2-hydroxyethylamino)-anisol, 2-(2,4-Diaminophenoxy)-ethanol, 3-Amino-2,4-dichlorphenol, 2-Methyl-5-aminophenol, 3-Methyl-4-aminophenol, 2-Methyl-5-(2-hydroxyethylamino)phenol, 2-Methyl-5-amino-4-chlorphenol, 6-Methyl-3-amino-2-chlorphenol, 2-Aminomethyl-4-aminophenol, 2-Diethylaminomethyl-4-aminophenol, 2-Dimethylaminomethyl-4-aminophenol, 2,6-Dichlor-4-aminophenol, 2-Hydroxymethyl-4-aminophenol, 2,6-Bis(2-hydroxyethylamino)-1-methylbenzol, Bis-(2-hydroxy-5-aminophenyl)methan, Bis-(4,5-amino-2-hydroxyphenyl)-methan, 1,3-Bis(2,4-diaminophenoxy)propan, 1,4-Bis(4-aminophenyl)-1,4-diazacycloheptan, 1,8-Bis (2,5-Diaminophenoxy)-3,6-dioxaoctan, 4,4'-Diaminodiphenylamin, 3,4-Methylendioxyphenol, 3,4-Diaminobenzoesäure, 2,5-Diaminopyridin, 2-Dimethylamino-5-aminopyridin, 2-Amino-3-hydroxypyridin, 3-Amino-2-methylamino-6-methoxypyridin, 2,3-Diamino-6-methoxypyridin, 3,5-Diamino-2,6-dimethoxypyridin, 2,6-Dihydroxy-3,4-dimethylpyridin, 2-Hydroxy-4,5,6-triaminopyrimidin, 4-Hydroxy-2,5,6-triaminopyrimidin, 2,4,5,6-Tetraaminopyrimidin, 2-Methylamino-4,5,6-triamino-pyrimidin, 3,5-Diaminopyrazol, 3-Amino-5-hydroxypyrazol, 4,5-Diamino-1-(2-hydroxyethyl)-pyrazol, 5,6-Dihydroxyindol, 5,6-Dihydroxyindolin, 4-Amino-2,3-dimethyl-1-phenyl-3-pyrazolin-5-on (4-Aminoantipyrin), 2,3-Dimethylbenzothiazolium-p-toluolsulfonat, 1,2,3,3-Tetramethyl-3H-indolinium-p-toluolsulfonat, Thiobarbitursäure, Rhodanin, 2,3-Dimethyl-benzothiazolium-p-toluolsulfonat, 1,2-Dimethyl-naphtho[1,2-d]thiazolium-ptoluolsulfonat, 1-Methyl-2-chinaldinium-p-toluolsulfonat, 1,4-Dimethylchinolinium-p-toluolsulfonat, β-Alanin, L-Prolin, L-Lysin, DL-Tyrosin sowie jeweils aus den vorzugsweise mit anorganischen Säuren gebildeten physiologisch verträglichen Salzen dieser Verbindungen.

6. Mittel nach einem der Ansprüche 1 bis 5, **dadurch gekennzeichnet, daß** es Farbverstärker ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Arginin, Histidin, Pyrrolidin, Prolin, Pyrrolidon, Pyrrolidon-5-carbonsäure, Pyrazol, 1,2,4-Triazol, Piperazidin oder deren beliebigen Gemischen enthält.

7. Mittel nach einem der Ansprüche 1 bis 6, **dadurch gekennzeichnet, daß** es direkt ziehende Farbstoffe aus der Gruppe der Nitrophenylendiamine, Nitroaminophenole, Anthrachinone oder Indophenole vorzugsweise in einer Menge von 0,01 bis 20 Gew.-%, bezogen auf das gesamte Färbemittel, enthält.

8. Mittel nach einem der Ansprüche 1 bis 7, **dadurch gekennzeichnet, daß** Ammonium- oder Metallsalze ausgewählt aus der Gruppe der Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, zugegeben werden.

9. Mittel nach einem der Ansprüche 1 bis 8, **dadurch gekennzeichnet, daß** es Oxidationsmittel, insbesondere H₂O₂, in einer Menge von 0,01 bis 6 Gew.-%, bezogen auf die Anwendungslösung, enthält.

10. Mittel nach einem der Ansprüche 1 bis 9, **dadurch gekennzeichnet, daß** es anionische, zwitterionische oder nichtionische Tenside enthält.

11. Verwendung einer Verbindung gemäß Anspruch 1 in Kombination mit einer Komponente B gemäß Anspruch 1 als eine färbende Komponente in Haarfärbemitteln.

12. Verfahren zum Färben von keratinhaltigen Fasern, insbesondere menschlichen Haaren, worin ein Färbemittel, gemäß Anspruch 1, sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

## Revendications

1. Agents pour la teinture de fibres kératiniques, en particulier de cheveux humains, contenant au moins un dérivé de la 2-arylidèneindolin-3-one répondant à la formule 1 et/ou un de ses sels physiologiquement acceptables 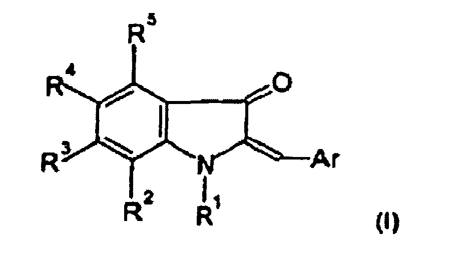 formule dans laquelle
• R¹ représente un atome d'hydrogène, un groupe alkyle en C₁-C₄, un groupe alcényle en C₂-C₆, un groupe aryle, un groupe arylalkyle en C₁-C₄, un groupe acyle en C₁-C₄, un groupe hydroxyalkyle en C₁₋C₄, un groupe dihydroxyalkyle en C₂-C₄, un groupe sulfo alkyle en C₁-C₄, un groupe carboxyalkyle en C₁-C₄, un groupe R^{I}R^{II}N-(CH₂)ₘ- où R^{I} et R^{II} représentent, indépendamment l'un de l'autre, un atome d'hydrogène, un groupe alkyle en C₁-C₄, un groupe hydroxyalkyle en C₁-C₄ ou un groupe arylalkyle en C₁-C₄, R^{I} et R^{II} pouvant former ensemble avec l'atome d'azote un noyau à 5 membres, à 6 membres ou à 7 membres, et m représente un nombre égal à 1, 2, 3 ou 4 ;
• R², R³, R⁴ et R⁵ représentent, indépendamment l'un de l'autre, un atome d'hydrogène, un atome d'halogène, un groupe alkyle en C₁₋C₄, un groupe alcoxy en C₁-C₄, un groupe hydroxyalcoxy en C₁-C₄, un groupe hydroxyle, un groupe nitro, un groupe carboxyle, un groupe d'acide sulfonique, un groupe sulfamoyle, un groupe R^{III}R^{IV}N-(CH₂)_{q}- où R^{III} et R^{IV} représentent, indépendamment l'un de l'autre, un atome d'hydrogène, un groupe alkyle en C₁-C₄, un groupe hydroxyalkyle en C₁-C₄ ou un groupe arylalkyle en C₁-C₄ et q représente un nombre égal à 0, 1, 2, 3 ou 4, deux des radicaux R², R³, R⁴ et R⁵ pouvant également former ensemble avec le noyau benzénique du corps de base de 2-arylidèneindolin-3-one un noyau carbocyclique ou hétérocyclique, aliphatique ou aromatique, à 5 membres ou à 6 membres ; et
• Ar représente un groupe phényle, un groupe naphtyle, un groupe styryle, un groupe pyridyle, un groupe pyrimidyle, un groupe pyrazyle, un groupe pyrazidyle, un groupe pyrrolyle, un groupe furyle, un groupe thiényle, un groupe 1,2,3-triazinyle, un groupe 1,3,5-triazinyle, un groupe quinolyle, un groupe isoquinolyle, un groupe indolyle, un groupe indolinyle, un groupe 3-oxoindolinyle, un groupe indolizinyle, un groupe indanyle, un groupe imidazolyle, un groupe 1,2,4-triazolyle, un groupe 1,2,3-triazolyle, un groupe tétrazolyle, un groupe benzimidazolyle, un groupe 1,3-thiazolyle, un groupe benzothiazolyle, un groupe indazolyle, un groupe benzoxazolyle, un groupe quinoxalinyle, un groupe quinazolinyle et un groupe cinnolinyle, le radical Ar étant, le cas échéant, substitué par un atome d'halogène, un groupe alkyle en C₁-C₄, un groupe alcoxy en C₁-C₄, un groupe hydroxyalkyle en C₁-C₄, un groupe hydroxyle, un groupe carboxyle, un groupe nitro, un groupe d'acide sulfonique ou un groupe R^{V}R^{VI}N-(CH₂)ᵣ- où R^{V} et R^{VI} représentent, indépendamment l'un de l'autre, un atome d'hydrogène, un groupe alkyle en C₁-C₄, un groupe hydroxyalkyle en C₁-C₄ ou un groupe arylalkyle en C₁-C₄ et r représente un nombre égal à 0, 1, 2, 3 ou 4
et, en outre, à titre de composant B, au moins un composé choisi parmi le groupe comprenant (a) des composés contenant un groupe amino primaire ou secondaire ou bien un groupe hydroxyle, choisi parmi le groupe comprenant des composés hydroxylés aromatiques, des amines aromatiques primaires ou secondaires et des composés hétérocycliques azotés, (b) des acides aminés et (c) des composés acides contenant un groupe CH.

2. Agent selon la revendication 1, **caractérisé en ce que** les dérivés de la 2-arylidèneindolin-3-one répondant à la formule I sont choisis parmi le groupe comprenant la 2-benzylidèneindolin-3-one, la 2-(2-hydroxybenzylidène)indolin-3-one, la 2-(4-hydroxybenzylidène)indolin-3-one, la 2-(4-méthoxybenzylidène)indolin-3-one, la 2-(4-méthoxybenzylidène)-5-nitroindolin-3-one, la 2-(4-nitrobenzylidène)-4-nitro-indolin-3-one, la 2-(4-nitrobenzylidène)-5-méthoxyindolin-3-one, la 2-(3-nitrobenzylidène)-indolin-3-one, la 2-(2-nitrobenzylidène)indolin-3-one, la 1-méthyl-2-(4-nitrobenzylidène)indolin-3-one, la 1-méthyl-2-(3-nitrobenzylidène)indolin-3-one, la 1-méthyl-2-(2-nitrobenzylidène)indolin-3-one, la 2-(3,4-diméthoxybenzylidène)indolin-3-one, la 2-(3,4-méthylènedioxybenzylidène)indolin-3-one, la 2-(4-diméthylaminobenzylidène)indolin-3-one, la 2-(2-furylméthylène)indolin-3-one, la 2-(2-thiénylméthylène)-indolin-3-one, la 2-(3-méthoxy-4-hydroxybenzylidène)indolin-3-one, la 2-(4-pyridylméthylène)indolin-3-one, la 5-bromo-2-(4-pyridylméthylène)-indolin-3-one, la 2-(2-pyridylméthylène)indolin-3-one, la 2-(2-quinolylméthylène)indolin-3-one, la 2-(4-quinolylméthylène)indolin-3-one, l'iodure du 2-(3-oxo-2-indolinylidèneméthyl)-1-méthylpyridinium, l'iodure du 4-(1-méthyl-3-oxo-2-indolinylidèneméthyl)-1-méthylpyridinium, l'iodure du 2-(1-méthyl-3-oxo-2-indolinylidèneméthyl)-1-méthylpyridinium, le méthylsulfonate du 4-(3-oxo-2-indolinylidèneméthyl)-1-méthylquinolinium, le p-toluènesulfonate du 4-(3-oxo-2-indolinylidèneméthyl)-1-méthylquinolinium, la 2-(2-pyrrolylméthylène)indolin-3-one, l'iodure du 4-(1-méthyl-3-oxo-2-indolinylidèneméthyl)-1-méthylpyrrolium, ainsi que leurs sels physiologiquement acceptables.

3. Agent selon l'une quelconque des revendications 1 ou 2, **caractérisé en ce que** les composés répondant à la formule I et les composés du composant B sont contenus respectivement en une quantité de 0,03 à 65 millimoles, en particulier de 1 à 40 millimoles, rapportés à 100 g de la teinture totale.

4. Agent selon l'une quelconque des revendications 1 à 3, **caractérisé en ce que** le composé du composant B est choisi parmi le groupe constitué par la N,N-diméthyl-p-phénylènediamine, la N,N-diéthyl-p-phénylènediamine, la N-(2-hydroxyéthyl)-N-éthyl-p-phénylènediamine, la N,N-bis-(2-hydroxyéthyl)-p-phénylènediamine, la N-(2-méthoxyéthyl)-p-phénylènediamine, la 2,3-dichloro-p-phénylènediamine, la 2,4-dichloro-p-phénylènediamine, la 2,5-dichloro-p-phénylènediamine, la 2-chloro-p-phénylènediamine, la 2,5-dihydroxy-4-morpholinoaniline, le 2-aminophénol, le 3-aminophénol, le 4-aminophénol, le 2-aminométhyl-4-aminophénol, le 2-hydroxyméthyl-4-aminophénol, la o-phénylènediamine, la m-phénylènediamine, la p-phénylènediamine, le 2,5-diaminotoluène, le 2,5,-diaminophénol, le 2,5-diaminoanisole, le 2,5-diaminophénéthol, le 4-amino-3-méthylphénol, le 2-(2,5-diaminophényl)-éthanol, le 2,4-diaminophénoxyéthanol, le 2-(2,5-diaminophénoxy)-éthanol, le 3-amino-4-(2-hydroxyéthyloxy)phénol, le 3,4-méthylènedioxyphénol, la 3,4-méthylènedioxyaniline, le 3-amino-2,4-dichlorophénol, le 4-méthylaminophénol, le 2-méthyl-5-aminophénol, le 3-méthyl-4-aminophénol, le 2-méthyl-5-(2-hydroxyéthylamino)phénol, le 3-amino-2-chloro-6-méthylphénol, le 2-méthyl-5-amino-4-chlorophénol, le 5-(2-hydroxyéthylamino)-4-méthoxy-2-méthylphénol, le 4-amino-2-hydroxyméthylphénol, le 2-(diéthylaminométhyl)-4-aminophénol, le 4-amino-1-hydroxy-2-(2-hydroxyéthylaminométhyl)benzène, le 1-hydroxy-2-amino-5-méthylbenzène, le 1-hydroxy-2-amino-6-méthylbenzène, le 2-amino-5-acétamidophénol, le 1,3-diméthyl-2,5-diaminobenzène, le 5-(3-hydroxypropylamino-)2-méthylphénol, le 5-amino-4-méthoxy-2-méthylphénol, le N,N-diméthyl-3-aminophénol, le N-cyclopentyl-3-aminophénol, le 5-amino-4-fluoro-2-méthylphénol, le 2,4-diamino-5-fluorotoluène, le 2,4-diamino-5-(2-hydroxyéthoxy)toluène, le 2,4-diamino-5-méthylphénéthol, le 3,5-diamino-2-méthoxy-1-méthylbenzène, le 2-amino-4-(2-hydroxyéthylamino]anisole, le 2,6-bis-(2-hydroxyéthylamino)-1-méthylbenzène, le 1,3-diamino-2,4-diméthoxybenzène, le 3,5-diamino-2-méthoxytoluène, l'acide 2-aminobenzoïque, l'acide 3-aminobenzoïque, l'acide 4-aminobenzoïque, l'acide 2-aminophénylacétique, l'acide 3-aminophénylacétique, l'acide 4-aminophénylacétique, l'acide 2,3-diaminobenzoïque, l'acide 2,4-diaminobenzoïque, l'acide 2,5-diaminobenzoïque, l'acide 3,4-diaminobenzoïque, l'acide 3,5-diaminobenzoïque, l'acide 4-aminosalicylique, l'acide 5-aminosalicylique, l'acide 3-amino-4-hydroxybenzoïque, l'acide 4-amino-3-hydroxybenzoïque, l'acide 2-aminobenzènesulfonique, l'acide 3-aminobenzènesulfonique, l'acide 4-aminobenzènesulfonique, l'acide 3-amino-4-hydroxybenzènesulfonique, l'acide 4-amino-3-hydroxynaphtalène-1-sulfonique, l'acide 6-amino-7-hydroxynaphtalène-2-sulfonique, l'acide 7-amino-4-hydroxynaphtalène-2-sulfonique, l'acide 4-amino-5-hydroxy-naphtalène-2,7-disulfonique, l'acide 3-amino-2-naphtoïque, l'acide 3-aminophtalique, l'acide 5-aminoisophtalique, le 1,3,5-triaminobenzène, le 1,2,4-triaminobenzène, le 1,2,4,5-tétraaminobenzène, le 2,4,5-triaminophénol, le pentaaminobenzène, l'hexaaminobenzène, le 2,4,6-triaminorésorcinol, le 4,5-diaminopyrocatéchol, le 4,6-diaminopyrogallol, la 1-(2-hydroxy-5-aminobenzyl)-2-imidazolidinone, le 4-amino-2-((4-[(5-amino-2-hydroxyphényl)méthyl]-pipérazinyl)méthyl)phénol, le 3,5-diamino-4-hydroxypyrocatéchol, le 1,4-bis-(4-aminophényl]-1,4-diazacycloheptane, des nitriles aromatiques tels que le 2-amino-4-hydroxybenzonitrile, le 4-amino-2-hydroxybenzonitrile, le 4-aminobenzonitrile, le 2,4-diaminobenzonitrile, des composés amino contenant un ou plusieurs groupes nitro, tels que la 3-amino-6-méthylamino-2-nitropyridine, l'acide picramique, le chlorure du [8-[(4-amino-2-nitrophényl)-azo]-7-hydroxynapht-2-yl]triméthylammonium, le chlorure du (8-((4-amino-3-nitrophényl)-azo)-7-hydroxy-napht-2-yl]diméthylammonium (Basic Brown 17), le 1-hydroxy-2-amino-4,6-dinitrobenzène, le 1-amino-2-nitro-4-[bis-(2-hydroxyéthyl)amino]benzène, le 1-amino-2-[(2-hydroxyéthyl)amino]-5-nitrobenzène (HC Yellow n° 5), le 1-amino-2-nitro-4-[(2-hydroxyéthyl)amino]benzène (HC Red n° 7), la 2-chloro-5-nitro-N-hydroxyéthyl-1,4-phénylènediamine, le 1-(2'-hydroxyéthyl)-amino-2-nitro-4-aminobenzène (HC Red n° 3), le 4-amino-3-nitrophénol, le 4-amino-2-nitrophénol, la 6-nitro-o-toluidène, le 1-amino-3-méthyl-4-(2'-hydroxyéthyl)amino-6-nitrobenzène (HC Violet n° 1), le 1-amino-2-nitro-4-(2',3'-dihydroxypropyl)-amino-5-chlorobenzène (HC Red n° 10), l'acide 2-(4'-amino-2'-nitroanilino)benzoïque, la 6-nitro-2,5-diaminopyridine, le 2-amino-6-chloro-4-nitrophénol, le sel disodique de l'acide 1-amino-2-(3'-nitrophénylazo)-7-phénylazo-8-naphtol-3,6-disulfonique (Acid blue n° 29), le sel disodique de l'acide 1-amino-2-(2-hydroxy-4-nitrophénylazo)-8-naphtol-3,6-disulfonique (vert de palatin-chrome), le sel disodique de l'acide 1-amino-2-(3-chloro-2-hydroxy-5-nitrophénylazo)-8-naphtol-3,6-disulfonique (gallion), le sel disodique de l'acide 4-amino-4'-nitrostilbène-2,2'-disulfonique, le 2,4-diamino-3',5'-dinitro-2'-hydroxy-5-méthylazobenzène (Mordant brown 4), l'acide 4'-amino-4-nitrodiphénylamine-2-sulfonique, l'acide 4'-amino-3'-nitrobenzophénone-2-carboxylique, le 1-amino-4-nitro-2-(2'-nitrobenzylidèneamino)benzène, la 2-[2'-(diéthylamino)éthylamino]-5-nitroaniline, l'acide 3-amino-4-hydroxy-5-nitrobenzènesulfonique, le 3-amino-3'-nitrobiphényle, le 3-amino-4-nitroacénaphtène, le 2-amino-1-nitronaphtalène, le 5-amino-6-nitrobenzo-1,3-dioxol, des anilines, en particulier des anilines contenant des groupes nitro, telles que la 4-nitroaniline, la 2-nitroaniline, le 1,4-diamino-2-nitrobenzène, le 1,2-diamino-4-nitrobenzène, le 1-amino-2-méthyl-6-nitrobenzène, la 4-nitro-1,3-phénylènediamine, le 2-nitro-4-amino-1-(2-hydroxyéthylamino)benzène, le 2-nitro-1-amino-4-[bis-(2-hydroxyéthyl)-amino]benzène, l'acide 4-amino-2-nitrodiphénylamine-2'-carboxylique, le 1-amino-5-chloro-4-(2'-hydroxyéthylamino)-2-nitrobenzène, le 4,4'-diaminostilbène et son chlorhydrate, le sel monosodique ou disodique de l'acide 4,4'-diaminostilbène-2,2'-disulfonique, le 4-amino-4'-diméthylaminostilbène et son chlorhydrate, le 4,4'-diaminodiphénylméthane, le 4,4'-diaminodiphénylsulfure, le 4,4'-diaminodiphénylsulfoxyde, la 4,4'-diaminodiphénylamine, l'acide 4,4'-diaminodiphénylamine-2-sulfonique, la 4,4'-diaminobenzophénone, l'éther 4,4'-diaminodiphénylique, le 3,3',4,4'-tétra-aminodiphényle, la 3,3',4,4'-tétra-aminobenzophénone, le 1,3-bis-(2,4-diaminophénoxy)propane, le 1,8-bis-(2,5-diaminophénoxy)-3,6-dioxaoctane, le 1,3-bis-(4-aminophénylamino)propane, le 1,8-bis-(4-aminophénylamino)-2-propanol, le 1,3-bis-[N-(4-aminophényl)-2-hydroxyéthylamino]-2-propanol, la N,N-bis-[2-(4-aminophénoxy)éthyl]-méthylamine, la N-phényl-1,4-phénylènediamine et le bis-(5-amino-2-hydroxyphényl)méthane, des composés hétérocycliques azotés choisis parmi le groupe constitué par la 2-aminopyridine, la 3-aminopyridine, la 4-aminopyridine, la 2-amino-3-hydroxypyridine, la 2,6-diaminopyridine, la 2,5-diaminopyridine, la 2-(aminoéthylamino)-5-aminopyridine, la 2,3-diaminopyridine, la 2-diméthylamino-5-aminopyridine, la 2-méthylamino-3-amino-6-méthoxypyridine, la 2,3-diamino-6-méthoxypyridine, la 2,6-diméthoxy-3,5-diaminopyridine, la 2,4,5-triaminopyridine, la 2,6-dihydroxy-3,4-diméthylpyridine, la N-[2-(2,4-diaminophényl)aminoéthyl]-N-(5-amino-2-pyridyl)amine, la N-[2-(4-aminophényl)aminoéthyl]-N-(5-amino-2-pyridyl)amine, la 2,4-dihydroxy-5,6-diaminopyrimidine, la 4,5,6-triaminopyrimidine, la 4-hydroxy-2,5,6-triaminopyrimidine, la 2-hydroxy-4,5,6-triaminopyrimidine, la 2,4,5,6-tétraaminopyrimidine, la 2-méthylamino-4,5,6-triaminopyrimidine, la 2,4-diaminopyrimidine, la 4,5-diaminopyrimidine, la 2-amino-4-méthoxy-6-méthylpyrimidine, le 3,5-diaminopyrazole, le 3,5-diamino-1,2,4-triazole, le 3-aminopyrazole, le 3-amino-5-hydroxypyrazole, le 1-phényl-4,5-diaminopyrazole, le 1-(2-hydroxyéthyl)-4,5-diaminopyrazole, le 1-phényl-3-méthyl-4,5-diaminopyrazole, la 4-amino-2,3-diméthyl-1-phényl-3-pyrazolin-5-one (la 4-aminoantipyrine), la 1-phényl-3-méthylpyrazol-5-one, la 2-aminoquinoléine, la 3-aminoquinoléine, la 8-aminoquinoléine, la 4-aminoquinaldine, l'acide 2-aminonicotinique, l'acide 6-aminonicotinique, la 5-aminoisoquinoléine, le 5-aminoindazole, le 6-aminoindazole, le 5-aminobenzimidazole, le 7-aminobenzimidazole, le 5-aminobenzothiazole, le 7-aminobenzothiazole, la 2,5-dihydroxy-4-morpholinoaniline, ainsi que des dérivés d'indole et d'indoline, tels que le 4-aminoindole, le 5-aminoindole, le 6-aminoindole, le 7-aminoindole, le 5,6-dihydroxyindole, la 5,6-dihydroxyindoline, la 4-hydroxyindoline, des dérivés d'hydroxypyrimidine, ainsi qua des composés hydroxylés aromatiques tels que le 2-méthylrésorcinol, le 4-méthylrésorcinol, le 5-méthylrésorcinol, le 2,5-diméthylrésorcinol, le résorcinol, le 3-méthoxyphénol, le pyrocatéchol, l'hydroquinone, le pyrogallol, la phloroglucine, l'hydroxyhydroquinone, le 2-méthoxyphénol, le 3-méthoxyphénol, le 4-méthoxyphénol, le 3-diméthylaminophénol, le 2-(2-hydroxyéthyl)phénol, le 3,4-méthylènedioxyphénol, l'acide 2,4-dihydroxybenzoïque, l'acide 3,4-dihydroxybenzoïque, l'acide 2,4-dihydroxyphénylacétique, l'acide 3,4-dihydroxyphénylacétique, l'acide gallique, l'acide 2,4,6-trihydroxybenzoïque, la 2,4,6-trihydroxyacetophénone, le 2-chlororésorcinol, le 4-chlororésorcinol, le 1-naphtol, le 1,5-dihydroxynaphtalène, le 2,3-dihydroxynaphtalène, le 2,7-dihydroxynaphtalène, l'acide 6-diméthylamino-4-hydroxy-2-naphtalènesulfonique et l'acide 3,6-dihydroxy-2,7-naphtalènesulfonique, des acides aminés choisis parmi le groupe comprenant l'arginine, l'histidine, la tyrosine, la phénylalanine, la DOPA (la dihydroxyphénylalanine), l'ornithine, la proline, la lysine, le tryptophane, l'acide 6-aminocaproïque et la β-alanine, des composés acides contenant un groupe CH choisis parmi le groupe comprenant l'iodure du 1,2,3,3-tétraméthyl-3H-indolium, le p-toluènesulfonate du 1,2,3,3-tétraméthyl-3H-indolium, le méthanesulfonate du 1,2,3,3-tétraméthyl-3H-indolium, la 1,3,3-triméthyl-2-méthylèneindoline (base de Fischer), l'iodure du 2,3-diméthylbenzothiazolium, le p-toluènesulfonate du 2,3-diméthylbenzothiazolium, le p-toluènesulfonate du 1,2-diméthylnaphto[1,2-d]thiazolium, le p-toluènesulfonate du 1-éthyl-2-méthylnaphto-[1,2-d]thiazolium, la rhodanine, l'acide rhodanine-3-acétique, l'iodure du 1-méthyl-2-quinaldinium, le p-toluènesulfonate du 1-méthyl-2-quinaldinium, l'iodure du 1-éthyl-2-quinaldinium, le p-toluènesulfonate du 1-éthyl-2-quinaldinium, l'iodure du 1,4-diméthylquinolinium, le p-toluènesulfonate du 1,4-diméthylquinolinium, l'acide barbiturique, l'acide thiobarbiturique, l'acide 1,3-diméthylthiobarbiturique, l'acide 1,3-diéthylthiobarbiturique, l'oxindole, le 3-indoxylacetate, la 2-coumaranone, la 5-hydroxycoumaranone, la 6-hydroxycoumaranone, la 1-méthyl-3-phényl-2-pyrazolinone, l'indane-1,3-dione, l'indan-1-one, le benzoylacétonitrile, le 1-dicyanométhylèneindane, le 1,3-bis(dicyanométhylène)-indane, la 3-dicyanométhylèneindan-1-one, la 1,3-diiminoisoindoline, le chlorhydrate de la 2-amino-4-imino-1,3-thiazoline, la 3-cyano-1,4-diméthyl-6-hydroxy-2-pyridone et la 3-cyano-1-éthyl-6-hydroxy-4-méthyl-2-pyridone, ainsi que les sels physiologiquement acceptables de tous les composés susmentionnés.

5. Agent selon l'une quelconque des revendications 1 à 4, **caractérisé en ce que** le composant B est choisi parmi des composés faisant partie du groupe constitué par la N-(2-hydroxyéthyl)-N-éthyl-p-phénylènediamine, la 2-chloro-p-phénylènediamine, la N,N-bis-(2-hydroxyéthyl)-p-phénylènediamine, le 2-aminophénol, le 3-aminophénol, le 4-aminophénol, le 2-amino-6-chloro-4-nitrophénol, la p-phénylènediamine, le 2-(2,5-diaminophényl)éthanol, le 2,5-diaminotoluène, la 3,4-méthylènedioxyaniline, le 2-amino-4-(2-hydroxyéthylamino)anisole, le 2-(2,4-diaminophénoxy)éthanol, le 3-amino-2,4-dichlorophénol, le 2-méthyl-5-aminophénol, le 3-méthyl-4-aminophénol, le 2-méthyl-5-(2-hydroxyéthylamino)phénol, le 2-méthyl-5-amino-4-chlorophénol, le 6-méthyl-3-amino-2-chlorophénol, le 2-aminométhyl-4-aminophénol, le 2-diéthylaminométhyl-4-aminophénol, le 2-diméthylaminométhyl-4-aminophénol, le 2,6-dichloro-4-aminophénol, le 2-hydroxyméthyl-4-aminophénol, le 2,6-bis(2-hydroxyéthylamino)-1-méthylbenzène, le bis-(2-hydroxy-5-aminophényl)méthane, le bis-(4,5-amino-2-hydroxyphényl)-méthane, le 1,3-bis-(2,4-diaminophénoxy)propane, le 1,4-bis-(4-aminophényl)-1,4-diazacycloheptane, le 1,8-bis-(2,5-diaminophénoxy)-3,6-dioxaoctane, la 4,4'-diaminodiphénylamine, le 3,4-méthylènedioxyphénol, l'acide 3,4-diaminobenzoïque, la 2,5-diaminopyridine, la 2-diméthylamino-5-aminopyridine, la 2-amino-3-hydroxypyridine, la 3-amino-2-méthylamino-6-méthoxypyridine, la 2,3-diamino-6-méthoxypyridine, la 3,5-diamino-2,6-diméthoxypyridine, la 2,6-dihydroxy-3,4-diméthylpyridine, la 2-hydroxy-4,5,6-triaminopyrimidine, la 4-hydroxy-2,5,6-triaminopyrimidine, la 2,4,5,6-tétraaminopyrimidine, la 2-méthylamino-4,5,6-triaminopyrimidine, le 3,5-diaminopyrazole, le 3-amino-5-hydroxypyrazole, le 4,5-diamino-1-(2-hydroxyéthyl)pyrazole, le 5,6-dihydroxyindole, la 5,6-dihydroxyindoline, la 4-amino-2,3-diméthyl-1-phényl-3-pyrazolin-5-one (la 4-aminoantipyrine), le p-toluènesulfonate du 2,3-diméthylbenzothiazolium, le p-toluènesulfonate du 1,2,3,3-tetraméthyl-3H-indolinium, l'acide thiobarbiturique, la rhodanine, le p-toluènesulfonate du 2,3-diméthylbenzothiazolium, le p-toluènesulfonate du 1,2-diméthyl-naphto[1,2-d]thiazolium, le p-toluènesulfonate du 1-méthyl-2-quinaldinium, le p-toluènesulfonate du 1,4-diméthylquinolinium, la β-alanine, la L-proline, la L-lysine, la DL-tyrosine, ainsi que respectivement les sels physiologiquement acceptables de ces composés, formés de préférence avec des acides inorganiques.

6. Agent selon l'une quelconque des revendications 1 à 5, **caractérisé en ce qu'**il contient des agents de renforcement de colorants choisis parmi le groupe constitué par la pipéridine, l'acide pipéridine-2-carboxylique, l'acide pipéridine-3-carboxylique, l'acide pipéridine-4-carboxylique, la pyridine, la 2-hydroxypyridine, la 3-hydroxypyridine, la 4-hydroxypyridine, l'imidazole, le 1-méthylimidazole, l'arginine, l'histidine, la pyrrolidine, la proline, la pyrrolidone, l'acide pyrrolidone-5-carboxylique, le pyrazole, le 1,2,4-triazole, la pipérazidine ou l'un quelconque de leurs mélanges.

7. Agent selon l'une quelconque des revendications 1 à 6, **caractérisé en ce qu'**il contient des colorants montant directement sur la fibre choisis parmi le groupe comprenant des nitrophénylènediamines, des nitroaminophénols, des anthraquinones ou des indophénols, de préférence en une quantité de 0,01 à 20 % en poids, rapportés à la teinture totale.

8. Agent selon l'une quelconque des revendications 1 à 7, **caractérisé en ce qu'**on ajoute des sels d'ammonium ou des sels métalliques choisis parmi le groupe comprenant des formiates, des carbonates, des halogénures, des sulfates, des butyrates, des valérates, des caproates, des acétates, des lactates, des glycollates, des tartrates, des citrates, des gluconates, des propionates, des phosphates et des phosphonates de métaux alcalins tels que le potassium, le sodium ou le lithium, de métaux alcalino-terreux tels que le magnésium, le calcium, le strontium ou le baryum ou encore de l'aluminium, du manganèse, du fer, du cobalt, du cuivre ou du zinc.

9. Agent selon l'une quelconque des revendications 1 à 8, **caractérisé en ce qu'**il contient des agents d'oxydation, en particulier du H₂O₂, en une quantité de 0,01 à 6 % en poids, rapportés à la solution d'utilisation.

10. Agent selon l'une quelconque des revendications 1 à 9, **caractérisé en ce qu'**il contient des agents tensioactifs anioniques, zwitterioniques ou non ioniques.

11. Utilisation d'un composé selon la revendication 1 en combinaison avec un composant B selon la revendication 1, à titre de composant de coloration dans des teintures capillaires.

12. Procédé pour la teinture de fibres kératiniques, en particulier de cheveux humains, dans lequel on applique une teinture selon la revendication 1, ainsi que des constituants cosmétiques habituels sur les fibres kératiniques, on les laisse pendant un certain temps, habituellement pendant 30 minutes, sur les fibres, avant de les en éliminer par rinçage ou par lavage avec un shampooing.

## Claims

1. Agent for dyeing keratinous fibres, in particular human hair, comprising at least one 2-arylideneindolin-3-one derivative according to formula I and/or physiologically compatible salts thereof, 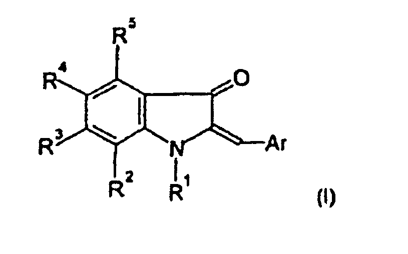 where
• R¹ is a hydrogen atom, a C₁-C₄-alkyl group, a C₂-C₆₋alkenyl group, an aryl group, an aryl-C₁-C₄-alkyl group, a C₁-C₄-acyl group, a C₁-C₄-hydroxyalkyl group, a C₂-C₄-dihydroxyalkyl group, a C₁-C₄-sulphoalkyl group, a C₁-C₄-carboxyalkylgroup, a group R^{I}R^{II}N-(CH₂)ₘ-, in which R^{I} and R^{II}, independently of one another, are a hydrogen atom, a C₁-C₄-alkyl group, a C₁-C₄-hydroxyalkyl group or an aryl-C₁-C₄- alkyl group, where R^{I} and R^{II}, together with the nitrogen atom, can form a 5-, 6- or 7-membered ring and m is a number 1, 2, 3 or 4,
• R², R³, R⁴ and R⁵, independently of one another, are a hydrogen atom, a halogen atom, a C₁-C₄-alkyl group, a C₁-C₄-alkoxy group, a C₁-C₄-hydroxyalkoxy group, a hydroxy group, a nitro group, a carboxy group, a sulphonic acid group, a sulphamoyl group, a group R^{III}R^{IV}N- (CH₂) _{q}-, in which R^{III} and R^{IV}, independently of one another, are a hydrogen atom, a C₁-C₄-alkyl group, a C₁-C₄-hydroxyalkyl group or an aryl-C₁-C₄₋alkyl group and q is a number 0, 1, 2, 3 or 4, where also two of the radicals R², R³, R⁴ and R⁵, together with the benzene ring of the 2-arylidene-indolin-3-one basic structure, can form a 5- or 6-membered, carbocyclic or heterocyclic, aliphatic or aromatic ring, and
• Ar is phenyl, naphthyl, styryl, pyridyl, pyrimidyl, pyrazyl, pyrazidyl, pyrrolyl, furyl, thienyl, 1,2,3-triazinyl, 1,3,5-triazinyl, quinolyl, isoquinolyl, indolyl, indolinyl, 3-oxoindolinyl, indolizinyl, indanyl, imidazolyl, 1,2,4-triazolyl, 1,2,3-triazolyl, tetrazolyl, benzimidazoyl, 1,3-thiazolyl, benzothiazolyl, indazolyl, benzoxazolyl, quinoxalinyl, quinazolinyl and cinnolinyl, where the Ar radical is optionally substituted by a halogen atom, a C₁-C₄-alkyl group, a C₁-C₄-alkoxy group, a C₁₋C₄-hydroxyalkyl group, a hydroxy group, a carboxy group, a nitro group, a sulphonic acid group or a group R^{V}R^{VI}N- (CH₂)ᵣ-, in which R^{V} and R^{VI}, independently of one another are a hydrogen atom, a C₁-C₄-alkyl group, a C₁-C₄-hydroxyalkyl group or an aryl-C₁-C₄-alkyl group and r is a number 0, 1, 2, 3 or 4
and additionally, as component B, at least one compound chosen from (a) compounds with primary or secondary amino or hydroxy group, chosen from aromatic hydroxy compounds, primary or secondary aromatic amines and nitrogen-containing heterocyclic compounds, (b) amino acids and (c) CH-acidic compounds.

2. Agent according to Claim 1, **characterized in that** the 2-arylidene-indolin-3-one derivatives of the formula I are chosen from 2-benzylideneindolin-3-one, 2-(2-hydroxybenzylidene)indolin-3-one, 2- (4-hydroxybenzylidene) indolin-3-one, 2-(4-methoxybenzylidene)-indolin-3-one, 2-(4-methoxybenzylidene)-5-nitroindolin-3-one, 2-(4-nitrobenzylidene)-4-nitroindolin-3-one, 2-(4-nitrobenzylidene)-5-methoxyindolin-3-one, 2-(3-nitrobenzylidene) indolin-3-one, 2- (2-nitrobenzylidene) indolin-3-one, 1-methyl-2-(4-nitrobenzylidene)indolin-3-one, 1-methyl-2-(3-nitrobenzylidene)indolin-3-one, 1-methyl-2-(2-nitrobenzylidene)indolin-3-one, 2-(3,4-dimethoxybenzylidene)indolin-3-one, 2-(3,4-methylenedioxybenzylidene)indolin-3-one, 2-(4-dimethylaminobenzylidene)indolin-3-one, 2-(2-furylmethylene) indolin-3-one, 2-(2-thienylmethylene)indolin-3-one, 2-(3-methoxy-4-hydroxybenzylidene)indolin-3-one, 2-(4-pyridylmethylene) indolin-3-one, 5-bromo-2-(4-pyridylmethylene)indolin-3-one, 2- (2-pyridylmethylene) indolin-3-one, 2-(2-quinolylmethylene)indolin-3-one, 2-(4-quinolylmethylene) indolin-3-one, 2- (3-oxo-2-indolinylidenemethyl) -1-methylpyridinium iodide, 4-(1-methyl-3-oxo-2-indolinylidenemethyl) -1-methylpyridinium iodide, 2-(1-methyl-3-oxo-2-indolinylidenemethyl)-1-methylpyridinium iodide, 4-(3-oxo-2-indolinylidenemethyl)-1-methylquinolinium methylsulphonate, 4-(3-oxo-2-indolinylidenemethyl)-1-methylquinolinium p-toluene sulphonate, 2-(2-pyrrolylmethylene)indolin-3-one, 4-(1-methyl-3-oxo-2-indolinylidenemethyl)-1-methylpyrrolium iodide, and physiologically compatible salts thereof.

3. Agent according to one of claims 1 or 2, **characterized in that** the compounds of the formula I and the compounds of component B are in each case present in an amount of from 0.03 to 65 mmol, in particular from 1 to 40 mmol, based on 100g of the total dyeing agent.

4. Agent according to one of Claims 1 to 3, **characterized in that** the compound of the component B is chosen from N,N-dimethyl-p-phenylenediamine, N,N-diethyl-p-phenylenediamine, N-(2-hydroxyethyl)-N-ethyl-p-phenylenediamine, N,N-bis(2-hydroxyethyl)-p-phenylenediamine, N-(2-methoxyethyl)-p-phenylenediamine, 2,3-dichloro-p-phenylenediamine, 2,4-dichloro-p-phenylenediamine, 2,5-dichloro-p-phenylenediamine, 2-chloro-p-phenylenediamine, 2,5-dihydroxy-4-morpholinoaniline, 2-aminophenol, 3-aminophenol, 4-aminophenol, 2-aminomethyl-4-aminophenol, 2-hydroxymethyl-4-aminophenol, o-phenylenediamine, m-phenylenediamine, p-phenylenediamine, 2,5-diaminotoluene, 2,5-diaminophenol, 2,5-diaminoanisole, 2,5-diamainophenetol, 4-amino-3-methylphenol, 2-(2,5-diaminophenyl)ethanol, 2,4-diaminophenoxyethanol, 2-(2,5-diaminophenoxy)ethanol, 3-amino-4-(2-hydroxyethyloxy)phenol, 3,4-methylenedioxyphenol, 3,4-methylenedioxyaniline, 3-amino-2,4-dichlorophenol, 4-methylaminophenol, 2-methyl-5-aminophenol, 3-methyl-4-aminophenol, 2-methyl-5-(2-hydroxyethylamino)phenol, 3-amino-2-chloro-6-methylphenol, 2-methyl-5-amino-4-chlorophenol, 5-(2-hydroxyethylamino)-4-methoxy-2-methylphenol, 4-amino-2-hydroxymethylphenol, 2-(diethylaminomethyl)-4-aminophenol, 4-amino-1-hydroxy-2-(2-hydroxyethylaminomethyl)benzene, 1-hydroxy-2-amino-5-methyl benzene, 1-hydroxy-2-amino-6-methyl benzene, 2-amino-5-acetamidophenol, 1,3-dimethyl-2,5-diaminobenzene, 5-(3-hydroxypropylamino)-2-methylphenol, 5-amino-4-methoxy-2-methylphenol, N,N-dimethyl-3-aminophenol, N-cyclopentyl-3-aminophenol, 5-amino-4-fluoro-2-methylphenol, 2,4-diamino-5-fluorotoluene, 2,4-diamino-5-(2-hydroxyethoxy) toluene, 2,4-diamino-5-methylphenetol, 3,5-diamino-2-methoxy-1-methylbenzene, 2-amino-4-(2-hydroxyethylamino)anisole, 2,6-bis(2-hydroxyethylamino)-1-methylbenzene, 1,3-diamino-2,4-dimethoxybenzene, 3,5-diamino-2-methoxytoluene, 2-aminobenzoic acid, 3-aminobenzoic acid, 4-aminobenzoic acid, 2-aminophenylacetic acid, 3-aminophenylacetic acid, 4-aminophenylacetic acid, 2,3-diaminobenzoic acid, 2,4-diaminobenzoic acid, 2,5-diaminobenzoic acid, 3,4-diaminobenzoic acid, 3,5-diaminobenzoic acid, 4-aminosalicylic acid, 5-aminosalicylic acid, 3-amino-4-hydroxybenzoic acid, 4-amino-3-hydroxybenzoic acid, 2-aminobenzenesulphonic acid, 3-aminobenzenesulphonic acid, 4-aminobenzenesulphonic acid, 3-amino-4-hydroxybenzenesulphonic acid, 4-amino-3-hydroxynaphthalene-1-sulphonic acid, 6-amino-7-hydroxynaphthalene-2-sulphonic acid, 7-amino-4-hydroxynaphthalene-2-sulphonic acid, 4-amino-5-hydroxynaphthalene-2,7-disulphonic acid, 3-amino-2-naphthoic acid, 3-aminophthalic acid, 5-aminoisophthalic acid, 1,3,5-triaminobenzene, 1,2,4-triaminobenzene, 1,2,4,5-tetraaminobenzene, 2,4,5-triaminophenol, pentaaminobenzene, hexaaminobenzene, 2,4,6-triaminoresorcinol, 4,5-diaminopyrocatechin, 4,6-diaminopyrogallol, 1-(2-hydroxy-5-aminobenzyl)-2-imidazolidinone, 4-amino-2-((4-[(5-amino-2-hydroxyphenyl) methyl] piperazinyl) methyl) phenol, 3,5-diamino-4-hydroxypyrocatechin, 1,4-bis(4-aminophenyl)-1,4-diazacycloheptane, aromatic nitriles, such as 2-amino-4-hydroxybenzonitrile, 4-amino-2-hydroxybenzonitrile, 4-aminobenzonitrile, 2,4-diaminobenzonitrile, amino compounds containing nitro groups, such as 3-amino-6-methylamino-2-nitropyridine, picramic acid, [8-[(4-amino-2-nitrophenyl)azo]-7-hydroxynaphth-2-yl]trimethylammonium chloride, [8-((4-amino-3-nitrophenyl)azo)-7-hydroxynaphth-2-yl]trimethylammonium chloride (Basic Brown 17), 1-hydroxy-2-amino-4,6-dinitrobenzene, 1-amino-2-nitro-4-[bis(2-hydroxyethyl)amino]benzene, 1-amino-2-[(2-hydroxyethyl)amino]-5-nitrobenzene (HC Yellow No. 5), 1-amino-2-nitro-4-[(2-hydroxyethyl)amino]benzene (HC Red No. 7), 2-chloro-5-nitro-N-2-hydroxyethyl-1,4-phenylenediamine, 1-[(2-hydroxyethyl)amino]-2-nitro-4-aminobenzene (HC Red No. 3), 4-amino-3-nitrophenol, 4-amino-2-nitrophenol, 6-nitro-o-toluidine, 1-amino-3-methyl-4-[(2-hydroxyethyl)amino]-6-nitrobenzene (HC Violet No. 1), 1-amino-2-nitro-4-[(2,3-dihydroxypropyl)amino]-5-chlorobenzene (HC Red No. 10), 2-(4-amino-2-nitroanilino)benzoic acid, 6-nitro-2,5-diaminopyridine, 2-amino-6-chloro-4-nitrophenol, 1-amino-2-(3-nitrophenylazo)-7-phenylazo-8-naphthol-3,6-disulphonic acid disodium salt (Acid blue No. 29), 1-amino-2-(2-hydroxy-4-nitrophenylazo)-8-naphthol-3,6-disulphonic acid disodium salt (Palatine chrome green), 1-amino-2-(3-chloro-2-hydroxy-5-nitrophenylazo)-8-naphthol-3,6-disulphonic acid disodium salt (Gallion), 4-amino-4'-nitrostilbene-2,2'-disulphonic acid disodium salt, 2,4-diamino-3'5'-dinitro-2'-hydroxy-5-methylazobenzene (Mordant brown 4), 4'-amino-4-nitrodiphenylamine-2-sulphonic acid, 4'-amino-3'-nitrobenzophenone-2-carboxylic acid, 1-amino-4-nitro-2-(2-nitrobenzylideneamino)benzene, 2-[2-(diethylamino)ethylamino]-5-nitroaniline, 3-amino-4-hydroxy-5-nitrobenzenesulphonic acid, 3-amino-3'-nitrobiphenyl, 3-amino-4-nitroacenaphthene, 2-amino-1-nitronaphthalene, 5-amino-6-nitrobenzo-1,3-dioxol, anilines, in particular anilines containing nitro groups, such as 4-nitroaniline, 2-nitroaniline, 1,4-diamino-2-nitrobenzene, 1,2-diamino-4-nitrobenzene, 1-amino-2-methyl-6-nitrobenzene, 4-nitro-1,3-phenylenediamine, 2-nitro-4-amino-1-(2-hydroxyethylamino)benzene, 2-nitro-1-amino-4-[bis(2-hydroxyethyl)amino]benzene, 4-amino-2-nitrodiphenylamine-2'-carboxylic acid, 1-amino-5-chloro-4-(2-hydroxyethylamino)-2-nitrobenzene, 4,4'-diaminostilbene and its hydrochloride, 4,4'-diaminostilbene-2,2'-disulphonic acid mono- or disodium salt, 4-amino-4'-dimethylaminostilbene and its hydrochloride, 4,4'-diaminodiphenylmethane, 4,4'-diaminodiphenyl sulphide, 4,4'-diaminodiphenyl sulphoxide, 4,4'-diaminodiphenylamine, 4,4'-diaminodiphenylamine-2-sulphonic acid, 4,4'-diaminobenzophenone, 4,4'-diaminodiphenylether, 3,3',4,4'-tetraaminodiphenyl, 3,3',4,4'-tetraaminobenzophenone, 1,3-bis(2,4-diaminophenoxy)propane, 1,8-bis(2,5-diaminophenoxy)-3,6-dioxaoctane, 1,3-bis(4-aminophenylamino)propane, 1,3-bis(4-aminophenylamino)-2-propanol, 1,3-bis[N-(4-aminophenyl)-2-hydroxyethylamino]-2-propanol, N,N-bis[2-(4-aminophenoxy)ethyl]methylamine, N-phenyl-1,4-phenylendiamine and bis(5-amino-2-hydroxyphenyl)methane, nitrogen-containing heterocyclic compounds chosen from the group consisting of 2-aminopyridine, 3-aminopyridine, 4-aminopyridine, 2-amino-3-hydroxypyridine, 2,6-diaminopyridine, 2,5-diaminopyridine, 2-(aminoethylamino)-5-aminopyridine, 2,3-diaminopyridine, 2-dimethylamino-5-aminopyridine, 2-methylamino-3-amino-6-methoxypyridine, 2,3-diamino-6-methoxypyridine, 2,6-dimethoxy-3,5-diaminopyridine, 2,4,5-triaminopyridine, 2,6-dihydroxy-3,4-dimethylpyridine, N-[2-(2,4-diaminophenyl)aminoethyl]-N-(5-amino-2-pyridyl)amine, N-[2-(4-aminophenyl)aminoethyl]-N-(5-amino-2-pyridyl)amine, 2,4-dihydroxy-5,6-diaminopyrimidine, 4,5,6-triaminopyrimidine, 4-hydroxy-2,5,6-triaminopyrimidine, 2-hydroxy-4,5,6-triaminopyrimidine, 2,4,5,6-tetraaminopyrimidine, 2-methylamino-4,5,6-triaminopyrimidine, 2,4-diaminopyrimidine, 4,5-diaminopyrimidine, 2-amino-4-methoxy-6-methylpyrimidine, 3,5-diaminopyrazole, 3,5-diamino-1,2,4-triazole, 3-aminopyrazole, 3-amino-5-hydroxypyrazole, 1-phenyl-4,5-diaminopyrazole, 1-(2-hydroxyethyl)-4,5-diaminopyrazole, 1-phenyl-3-methyl-4,5-diaminopyrazole, 4-amino-2,3-dimethyl-l-phenyl-3-pyrazolin-5-one(4-aminoantipyrine), 1-phenyl-3-methylpyrazol-5-one, 2-aminoquinoline, 3-aminoquinoline, 8-aminoquinoline, 4-aminoquinaldine, 2-aminocotinic acid, 6-aminonicotinic acid, 5-aminoisoquinoline, 5-aminoindazole, 6-aminoindazole, 5-aminobenzimidazole, 7-aminobenzimadazole, 5-aminobenzothiazole, 7-aminobenzothiazole, 2,5-dihydroxy-4-morpholinoaniline and indol and indoline derivates, such as 4-aminoindole, 5-aminoindole, 6-aminoindole, 7-aminoindole, 5,6-dihydroxyindole, 5,6-dihydroxyindoline, 4-hydroxyindoline, hydroxypyrimidine derivatives and aromatic hydroxy compounds, such as 2-methylresorcinol, 4-methylresorcinol, 5-methylresorcinol, 2,5-dimethylresorcinol, resorcinol, 3-methoxyphenol, pyrocatechin, hydroquinone, pyrogallol, phloroglucine, hydroxyhydroquinone, 2-methoxyphenol, 3-methoxyphenol, 4-methoxyphenol, 3-dimethylaminophenol, 2-(2-hydroxyethyl)phenol, 3,4-methylenedioxyphenol, 2,4-dihydroxybenzoic acid, 3,4-dihydroxybenzoic acid, 2,4-dihydroxyphenylacetic acid, 3,4-dihydroxyphenylacetic acid, gallic acid, 2,4,6-trihydroxybenzoic acid, 2,4,6-trihydroxyacetophenone, 2-chlororesorcinol, 4-chlororesorcinol, 1-naphthol, 1,5-dihydroxynaphthalene, 2,3-dihydroxynaphthalene, 2,7-dihydroxynaphthalene, 6-dimethylamino-4-hydroxy-2-naphthalenesulphonic acid and 3,6-dihydroxy-2,7-naphthalenesulphonic acid, amino acids chosen from the group consisting of arginine, histidine, tyrosine, phenylalanine, DOPA (dihydroxyphenylalanine), ornithine, proline, lysine, tryptophan, 6-aminocaproic acid and β-alanine, CH-acidic compounds chosen from the group consisting of 1,2,3,3-tetramethyl-3H-indolium iodide, 1,2,3,3-tetramethyl-3H-indolium p-toluenesulphonate, 1,2,3,3-tetramethyl-3H-indolium methanesulphonate, 1,3,3-trimethyl-2-methyleneindoline (Fischer's Base), 2,3-dimethylbenzothiazolium iodide, 2,3-dimethylbenzothiazolium p-toluenesulphonate, 1,2-dimethylnaphtho[1,2-d]thiazolium p-toluenesulphonate, 1-ethyl-2-methylnaphtho[1,2-d]thiazolium p-toluenesulphonate, rhodanine, rhodanine-3-acetic acid, 1-methyl-2-quinaldinium iodide, 1-methyl-2-quinaldinium p-toluenesulphonate, 1-ethyl-2-quinaldinium iodide, 1-ethyl-2-quinaldinium p-toluenesulphonate, 1,4-dimethylquinolinium iodide, 1,4-dimethylquinolinium p-toluenesulphonate, barbituric acid, thiobarbituric acid, 1,3-dimethylthiobarbituric acid, 1,3-diethylthiobarbituric acid, oxindole, 3-indoxyl acetate, 2-coumaranone, 5-hydroxycoumaranone, 6-hydroxycoumaranone, 1-methyl-3-phenyl-2-pyrazolinone, indane-1,3-dione, indan-1-one, benzoylacetonitrile, 1-dicyanomethyleneindane, 1,3-bis(dicyanomethylene)-indane, 3-dicyanomethyleneindan-1-one, 1,3-diiminoisoindoline, 2-amino-4-imino-1,3-thiazoline hydrochloride, 3-cyano-1,4-dimethyl-6-hydroxy-2-pyridone and 3-cyano-1-ethyl-6-hydroxy-4-methyl-2-pyridone, and the physiologically compatible salts of all of the abovementioned compounds.

5. Agent according to one of Claims 1 to 4, **characterized in that** the component B is chosen from compounds of the group consisting of N-(2-hydroxyethyl)-N-ethyl-p-phenylenediamine, 2-chloro-p-phenylenediamine, N,N-bis(2-hydroxyethyl)-p-phenylenediamine, 2-aminophenol, 3-aminophenol, 4-aminophenol, 2-amino-6-chloro-4-nitrophenol, p-phenylenediamine, 2-(2,5-diaminophenyl)ethanol, 2,5-diaminotoluene, 3,4-methylenedioxyaniline, 2-amino-4-(2-hydroxyethylamino)anisole, 2-(2,4-diaminophenoxy)ethanol, 3-amino-2,4-dichlorophenol, 2-methyl-5-aminophenol, 3-methyl-4-aminophenol, 2-methyl-5-(2-hydroxyethylamino)phenol, 2-methyl-5-amino-4-chlorophenol, 6-methyl-3-amino-2-chlorophenol, 2-aminomethyl-4-aminophenol, 2-diethylaminomethyl-4-aminophenol, 2-dimethylaminomethyl-4-aminophenol, 2,6-dichloro-4-aminophenol, 2-hydroxymethyl-4-aminophenol, 2,6-bis(2-hydroxyethylamino)-1-methylbenzene, bis(2-hydroxy-5-aminophenyl)methane, bis(4,5-amino-2-hydroxyphenyl)methane, 1,3-bis(2,4-diaminophenoxy)propane, 1,4-bis(4-aminophenyl)-1,4-diazacycloheptane, 1,8-bis(2,5-diaminophenoxy)-3,6-dioxaoctane, 4,4'-diaminodiphenylamine, 3,4-methylenedioxyphenol, 3,4-diaminobenzoic acid, 2,5-diaminopyridine, 2-dimethylamino-5-aminopyridine, 2-amino-3-hydroxypyridine, 3-amino-2-methylamino-6-methoxypyridine, 2,3-diamino-6-methoxypyridine, 3,5-diamino-2,6-dimethoxypyridine, 2,6-dihydroxy-3,4-dimethylpyridine, 2-hydroxy-4,5,6-triaminopyrimidine, 4-hydroxy-2,5,6-triaminopyrimidine, 2,4,5,6-tetraaminopyrimidine, 2-methylamino-4,5,6-triaminopyrimidine, 3,5-diaminopyrazole, 3-amino-5-hydroxypyrazole, 4,5-diamino-1-(2-hydroxyethyl)pyrazole, 5,6-dihydroxyindole, 5,6-dihydroxyindoline, 4-amino-2,3-dimethyl-1-phenyl-3-pyrazolin-5-one(4-aminoantipyrin), 2,3-dimethylbenzothiazolium p-toluenesulphonate, 1,2,3,3-tetramethyl-3H-indolinium p-toluenesulphonate, thiobarbituric acid, rhodanine, 2,3-dimethylbenzothiazolium p-toluenesulphonate, 1,2-dimethylnaphtho[1,2-d]thiazolium p-toluenesulphonate, 1-methyl-2-quinaldinium p-toluenesulphonate, 1,4-dimethylquinolinium p-toluenesulphonate, β-alanine, L-proline, L-lysine, DL-tyrosine, and in each case from the physiologically compatible salts of these compounds formed preferably with inorganic acids.

6. Agent according to one of Claims 1 to 5, **characterized in that** it comprises colour boosters chosen from the group consisting of piperidine, piperidine-2-carboxylic acid, piperidine-3-carboxylic acid, piperidine-4-carboxylic acid, pyridine, 2-hydroxypyridine, 3-hydroxypyridine, 4-hydroxypyridine, imidazole, 1-methylimidazole, arginine, histidine, pyrrolidine, proline, pyrrolidone, pyrrolidone-5-carboxylic acid, pyrazole, 1,2,4-triazole, piperazidine or any mixtures thereof.

7. Agent according to one of Claims 1 to 6, **characterized in that** it comprises direct dyes from the group of nitrophenylenediamines, nitroaminophenols, anthraquinones, or indophenols, preferably in an amount from 0.01 to 20% by weight, based on the total dyeing agent.

8. Agent according to one of Claims 1 to 7, **characterized in that** ammonium or metal salts chosen from the group of formates, carbonates, halides, sulphates, butyrates, valeriates, caproates, acetates, lactates, glycolates, tartrates, citrates, gluconates, propionates, phosphates and phosphonates of alkali metals, such as potassium, sodium or lithium, alkaline earth metals, such as magnesium, calcium, strontium or barium, or of aluminium, manganese, iron, cobalt, copper or zinc, are added.

9. Agent according to one of Claims 1 to 8, **characterized in that** it comprises oxidizing agents in particular H₂O₂, in an amount of from 0.01 to 6% by weight, based on the application solution.

10. Agent according to one of Claims 1 to 9, **characterized in that** it comprises anionic, zwitterionic or nonionic surfactants.

11. The use of a compound according to Claim 1 in combination with a component B according to Claim 1 as a dyeing component in hair dyeing agents.

12. Method of dyeing keratinous fibres, in particular human hair, in which a dyeing agent according to Claim 1, and customary cosmetic ingredients, are applied to the keratinous fibres, left on the fibres for a certain amount of time, usually about 30 minutes, and then rinsed out again or washed out with a shampoo.
